# EUROPEAN PATENT APPLICATION

(11) **EP 4 218 562 A1**
(43) Date of publication of application: **02.08.2023**
(21) Application number: 22153898.6
(22) Date of filing: 28.01.2022
(51) Int. Cl.: A61B 5/08, G01N 21/00, G01N 33/497

(54) **REAL-TIME BREATH ANTIGEN DETECTION SYSTEM AND METHOD THEREOF**

(71) Applicant: Charité - Universitätsmedizin Berlin, 10117 Berlin (DE)
(72) Inventor: Bierewirtz, Tim, 10965 Berlin (DE); Lommel, Michael, 10179 Berlin (DE); Kertzscher, Ulrich, 10713 Berlin (DE)
(74) Representative: Hertin und Partner Rechts- und Patentanwälte PartG mbB

(57) **Abstract**

The invention relates to a system for antigen detection comprising a first container configured to collect aerosols out of exhaled air of a human and a second container suitable for a solvent, wherein nanoparticles are dissolved in the solvent and the nanoparticles are linked to antibodies, further wherein a change of optical properties of the solvent is detectable upon contact between the antibodies and matching antigens. Here, a person can exhale air into the first container, and the aerosols exhaled in the air stick in a filter on one side of the first container. Thereupon, the first container is incooperable in the second container, in which a solvent with nanoparticles dissolved therein and antibodies coupled thereto are located. The incooperability, in particular a shuttling stroke motion, flushes the aerosols into the solvent. If antigens matching the antibodies are present on the aerosols, the nanoparticles agglomerate around the antigen, changing the optical properties of the solvent, which is irradiated by a light source and detectable by a light sensor.

In further aspects, the invention relates to a method using the system according to the invention.

The invention further relates to corresponding medical uses and therapeutic methods of administering a prevention of a medical condition associated with a SARS Coronavirus, in addition to diagnostic uses and methods.

## Description

The invention relates to a system for antigen detection comprising a first container configured to collect aerosols out of exhaled air of a human and a second container suitable for a solvent, wherein nanoparticles are dissolved in the solvent and the nanoparticles are linked to antibodies, further wherein a change of optical properties of the solvent is detectable upon contact between the antibodies and matching antigens. Here, a person can exhale air into the first container, and the aerosols exhaled in the air stick in a filter on one side of the first container. Thereupon, the first container is incooperable in the second container, in which a solvent with nanoparticles dissolved therein and antibodies coupled thereto are located. The incooperability, in particular a shuttling stroke motion, flushes the aerosols into the solvent. If antigens matching the antibodies are present on the aerosols, the nanoparticles agglomerate around the antigen, changing the optical properties of the solvent, which is irradiated by a light source and detectable by a light sensor.

In further aspects, the invention relates to a method using the system according to the invention.

The invention further relates to corresponding medical uses and therapeutic methods of administering a prevention of a medical condition associated with a SARS Coronavirus, in addition to diagnostic uses and methods.

### BACKGROUND OF THE INVENTION

Current studies demonstrate that human pathogens, especially viruses, can be transmitted by direct and indirect contact, but mainly by droplets and aerosols or particles in the inhalable size range (≤10 µm). Transmission via respirable particles (≤4 µm), which remain airborne for a long time and can be inhaled into the pulmonary alveoli, has been particularly controversial. As early as the 1940s, aerosol transmission between ferrets separated by up to 2.75 m was demonstrated. More recent animal studies support the proposition of airborne transmission (i.e., by aerosols and/or large droplets) over much shorter distances, from 5 cm to about 1 m, and this transmission was dependent on the species. The results of animal studies in which influenza viruses were experimentally transmitted by aerosols provide indirect evidence that transmission of viruses in populations may occur via the aerosol route. However, the sometimes large differences between animal species show that the transferability of the results to humans is questionable.

The Coronavirus (CoV) mediated respiratory disease was first observed in 2002. Once a human becomes infected, the virus can quickly spread via droplet transmission and close contact between humans, leading to epidemic scenarios or even to a pandemic. ⁽¹⁻³⁾

At present, the Corona pandemic is of great clinical importance to many people, especially those at high health risk. The impact on the economy represents another significant factor.

As one example, "Coronavirus Disease 2019" (COVID-19) is caused by the pathogenic corona virus SARS-CoV-2. Beginning in December 2019, the virus spread globally within a few weeks and led to an international health emergency. The worldwide pandemic poses huge challenges to health systems and leads additionally to restrictions in social life and weakening of global market economies. Since no effective therapy is available to date, and the disease is associated with high morbidity and mortality, there is a great need for therapeutic interventions for those who are ill as well as for prophylactic measures to contain outbreaks.^{(1,2)}

The predominant route of transmission of SARS-CoV-2, as currently understood, is via droplets and aerosols released into the ambient air by coughing, sneezing, breathing, or speaking from an infected person to a person who is in the process of becoming infected. The entry of SARS-CoV-2 into a cell is mediated by binding of the viral spike protein (S-protein) to the human angiotensin converting enzyme-2 (ACE2) target receptor.

The spread and possible infection via the airway are currently causing great anxiety among the people.

The transfer of viruses to respirable particles, which may be produced by coughing, sneezing and breathing, is a problem particularly in closed rooms and at large events with large groups of people, since the low level of air exchange allows these particles to remain in the air for a long period of time. If it were known quickly and reliably whether a person was expelling viruses and before they even entered a room and/or event or similar, it would be possible to avoid infecting other people and contain or even prevent the spread of the virus. ^{(1,4)}

Commercially available viral detection test systems, such as SARS-CoV-2, can identify viruses in the nasopharynx and/ or respiratory tract by swabbing. To evaluate a current or previous contact of a subject with a pathogen, specific antibodies in the mucosa of the nasalpharyngeal and/or respiratory tract are also screened, directly or indirectly, as mentioned. These known methods involve various problems that often coexist, such as the inconvenience to the subject of some sampling procedures; the provision of an analytical site; the possible pre-/treatment of the sample; the time required to perform the analysis; the use of reagents; the overall cost due to one or more of the previously mentioned known methods. However, the presence of viral material in swabs from mucous membranes is not necessarily an indication of a person's infectiousness, as it may be particles deposited there during inhalation, especially inhalation of virus-rich air, or viruses that have already been destroyed by local immune defenses and are therefore no longer active. This means, for example, that the material detected by the swabs of the known methods may be viruses that are already inactivated and may therefore give false-positive results. ^{(3,4)}

When an organism is infected by a pathogen, a particularly important variable is the number of infectious particles with which the organism comes into contact. However, the test systems with low sensitivity require a high viral load and have a high risk of false-negative results. The positive individual could be in different stages. The individual with high viral load is highly infectious, but usually already has symptoms by this time and likely stays at home. The affected individual can be paucisymptomatic or even asymptomatic, but may already be able to infect others. Individuals with a low yet infectious viral load, which may also increase during the course of an event, are not covered by this. Further, events and/or the gathering of people often span a period of several hours. It is well known that the viral load can also increase exponentially every hour. A negative test is no longer qualified for making a statement about the virus status of a person after only a short time. In addition, a meeting of a group of people would be problematic even with a low virus output, since on the one hand, it can increase and on the other hand, it can remain in the air and accumulate there.

These problems can limit the use of these methods, slow down the response time and consequently the intervention time, such as the isolation of the positive subject, thus contribute to the spread of the infectious agent and infection. ⁽⁵⁻⁶⁾

In the prior art, an emerging and so far unsolved problem is the long period between the test and the encounter of people and the low sensitivity of the test further disadvantage of the prior art is that the test systems are expensive and time-consuming to use and are not suitable for repeated measurement and/or measurement of large groups directly in advance of an event, at work or at school. Another unsolved problem is the environmental and economic disadvantages of the existing test systems. Further, the dyes used in the test system of the prior art can bleach out leading to false-negative results.

The problem solved by the invention is thus to develop a particularly sensitive, fast and reusable system and method to detect viruses and to reliably identify infected individuals.

A system and method for comprehensively and contemporaneous screening of exhaled air containing potentially infectious aerosols of a human in high-throughput is lacking. here is a high need in the general public, health care and education systems, as well as a high economic interest, of being able to reliably determine the infectious risk of any individual rapidly and in real-time.

The present invention provides a simple, fast, multi-use, sensitive and low-cost system that solves the problems in the prior art with one method.

### SUMMARY OF THE INVENTION

In light of the prior art, the technical problem underlying the invention was the provision of an alternative and simple system suitable to identify a human subject emitting a pathogen as an aerosol. More specifically, the problem underlying the invention is the provision of means for a rapid and sensitive detection of a pathogen using an antibody bound to a non-dye nanoparticle while avoiding dye breakdown and false results. Another problem underlying the invention is the provision of means for high throughput detection and quantification of pathogens in aerosols in exhaled air of a human subject.

A further problem to be solved by the underlying invention is the provision of a fast-track, optimized, low maintenance, and multi-use test system at lower cost while reducing the need for reagents and disposables.

This problem is solved by the features of the independent claims. Preferred embodiments of the present invention are provided by the dependent claims.

The invention therefore relates to a system for antigen detection comprising a first container configured to collect aerosols out of exhaled air of a human subject comprising aerosols and a second container suitable for a solvent, wherein nanoparticles are dissolved in the solvent and the nanoparticles are linked to antibodies, further wherein a change of optical properties of the solvent is detectable upon contact between the antibodies and matching antigens, wherein
- the system comprises a measuring cell and within the measuring cell there is a spectrometer comprising a light source and a light sensor
- and the first container comprises a hole at first side and a filter at second side
- and the second container is configured to incorporate the first container, wherein the filter of the first container contacts the solvent of the second container in the measuring cell,
- wherein during incorporating of the second container into the first container, a sealing between the shell surfaces of both containers forces the enclosed solvent in the second container to get pushed through the filter of the first container,
- an executable shuttling stroke motion between the first container and the second container flushes the filter and dissolves collected aerosols out of the filter into the solvent,
- wherein the light source irradiates the solvent and the light sensor is arranged to detect the change of optical properties
- and the system comprises an alarm device adapted to emit an alarm signal upon detection of the change of optical properties.

The combination of the present features of the invention lead to a surprising synergistic effect, resulting in the advantageous properties and the associated overall success of the invention, with the individual features interacting with each other.

The preferred system has proven to be particularly advantageous in many respects.

A particular advantage is that the preferred system enables a considerably higher sensitivity in the detection of antigens. This means that even the smallest amounts of down to 10 antigens can be detected in order to determine the infectivity of the exhaled air.

So, in addition, it is particularly advantageous to evaluate that the preferred system could produce a very low response time. Thus, it was recognized by the inventors that the preferred system could detect antigens on aerosols at a very high rate. Advantageously, results of a detection of antigens could be detected already after about 20 seconds, about 15 seconds, about 10 seconds, in particular also after about 5 seconds. The particularly fast measurement can be justified by the fact that the nanoparticles dissolved in the solvent with antibodies bound to them agglomerate particularly quickly, so that there is a change in optical properties and this change can be measured particularly quickly with the preferred arrangement of the spectroscope.

Furthermore, it is of great advantage that by means of the preferred system particularly reliable detection of antigens of the exhaled air is possible. Conventional tests, especially rapid tests, for example rapid tests for SARS-CoV-2 virus, tend to produce a certain rate of false-positive or false-negative tests. By means of the preferred system, the false-positive rate of prior art test results is significantly reduced, so that a considerable improvement has been achieved over the prior art.

Furthermore, it is of great advantage that the preferred system has efficient manageability. For example, the preferred system is of low mass and/or sizing so that it can be easily transported and easily operated. In preferred embodiments, the system is set up as a single unit, and it is preferred that the components of the preferred system interact and are operatively connected to each other. Preferably, the preferred system is arranged in, on, or to a support frame. In preferred embodiments, the preferred system has an overall unitary mass of up to about 5 kg, preferably up to about 2 kg, most preferably up to about 0.5 kg. In further preferred embodiments, the preferred system, preferably as a unit, has a length, width and/or height of up to about 1 m, preferably up to about 0.5 m, particularly preferably up to about 0.3 m, most preferably up to about 0.1 m. Due to the low mass and/or low dimensioning of the preferred system, the system according to the invention is advantageously suitable in a variety of applications.

For example, it is conceivable, in the context of leisure activities, to use the preferred system in a particularly simple manner to enable checks to be made as to whether or not a person has a certain load of antigens and thus a disease. In this way, large groups of people could be checked particularly quickly, efficiently and reliably, for example in the context of visits to discotheques, museums, restaurants, trains, etc.

It is also a particularly significant advantage that the preferred system is particularly easy to deploy. Thus, due to the preferred components of the system according to the invention, no complex manufacturing steps are required to set up the preferred system. Advantageously, complicated construction methods are eliminated compared to the prior art, preferably by the component such as the first container, the second container, the filter, the solvent, the light source and the light sensor. Advantageously, said components can be manufactured and/or acquired at low cost, so that the preferred system proves to be particularly efficient in the context of mass production and/or mass application.

The system according to the invention can thus be used to detect antigens on aerosols of exhaled air in a particularly efficient manner. This means that people can be tested positively or negatively for certain diseases. Due to its preferred design, the system according to the invention can be referred to as a passive puff system or passive puff test.

The present invention describes a method for detecting viruses or other pathogens (in particular, SARS-CoV-2 viruses) exhaled air of a human subject. Super-spreading events and vaccine breakthroughs can thus be contained or avoided completely.

At present, there is no rapid, real-time, multi-use, cost-effective and non-invasive antigen detection system available, particularly for detecting SARS-CoV-2 antigens that detects as low as 10 viral particles per sampling.

It is preferred that a human breathes or blows air into the first container. The exhaled air contains aerosols that enter the first filter during exhalation and in turn hit the filter of the first container and adhere to it.

The first container is then incorporated into the second container, where nanoparticles dissolved in a solvent are located inside the second container, on the surface of which antibodies are coupled. Preferably, the first container is incorporated into the second container by a shuttling stroke motion, so that the second container flushes the filter of the first container and now the solvent is guided into the first container and is present in contact with the aerosols. If antigens are present on the aerosols that match the antibodies of the nanoparticles, agglomeration of the nanoparticles occurs as antibodies and antigens combine with each other, causing a change in optical properties, which is detectable by a light sensor and a light source irradiating the solvent.

In some embodiments, the system comprises a computing unit, wherein the computing unit is configured to calculate a concentration of an antigen based on the change of optical properties of the solvent.

Advantageously, computing steps for measuring a concentration of antigens can be performed by the system. For this purpose, it is preferred that a computer program product, such as software and/or algorithms, is installed on the computing unit, which can execute corresponding computing operations. Advantageously, a result, in particular a precise result, about the concentrations of antigens can be determined by the system as such.

In some embodiments, the light source is a monochromatic laser comprising linearly polarized light, wherein said linearly polarized light is configured to excite pathogen-nanoparticle-agglomerates at extinction peak-wavelength, wherein said wavelengths preferably can be between 600 nm and 700nm, preferably between 610 nm and 690 nm, more preferably between 620 nm and 680 nm, even more preferably between 630 nm and 670 nm, even more between preferably 640 nm and 660 nm, even more preferably between 645 nm and 655 nm.

The use of a monochromatic laser is advantageous in that it has a particularly high photon density and is thus particularly well suited in the context of the invention to irradiate the solvents and aerosols dissolved therein by the introduction of the first container with sufficient intensity.

The wavelengths mentioned have thus proven to be particularly advantageous for enabling a particularly sensitive, especially precise and high-resolution measurement.

In some embodiments, the light sensor is preferably a spectral sensor, wherein preferably channels of the spectral sensor are configured to detect raman shifted light scattered by the pathogen-nanoparticle-agglomerates excited by the laser.

In some embodiments, the light sensor is mounted in line with the illumination direction of the light source to collect a high amount of the Mie-scattered light caused by the pathogen-nanoparticle-agglomerates, as those agglomerates scatter the most amount of light in the same direction as the incident light, resulting in an advantageously high measuring signal of the light sensor.

In further embodiments, a linear polarized filter is positioned in front of the light sensor to filter out the light and let through the depolarized light scattered by the pathogen-nanoparticle-agglomerates. As the depolarization-ratio of such pathogen-nanoparticle-agglomerates is higher than of other contaminated particles that the fluid might possibly contain, a better signal-to-noise-ratio is obtained by such filter.

In some embodiments, the alarm signal is selected from a group comprising a visual signal and/or an acoustic signal.

Advantageously, the signal types mentioned have proven to be particularly easy to implement. Furthermore, the signal types mentioned can clearly indicate to a user that a (critical) amount of antigen load has been detected.

In some embodiments, the system comprises a communication unit.

Advantageously, the communication unit can transmit a result of the measurement to an external data processing unit, such as a smartphone, a tablet and/or a computer. Thus, advantageously, multiple possibilities are offered to obtain the measurement result.

In some embodiments, the nanoparticles comprise a material selected from a group comprising gold, silver, ruthenium, rhodium, palladium, osmium, iridium and/or platinum and/or the nanoparticles might be spheres in the range of 15-25 nm or nanorods with a diameter of 10-25 nm and a length of 20-80 nm.

The aforementioned materials for the nanoparticles can be processed efficiently and cost-effectively in connection with the detection of antigens, in particular for binding the antibodies to the nanoparticles. Likewise, these materials are particularly well suited for further processing, for example in the context of further processing steps, in order to adapt them for binding to antibodies. In particular, desired radiation properties can thus be advantageously obtained.

The dimensions mentioned are relevant in that they depolarize the irradiated light. In particular, the degree of depolarization depends on the shape of the particles. An essentially round shape of the nanoparticles results in little depolarization of the light, whereas the rather irregular shape of the pathogen-nanoparticle-agglomerates results in a high depolarization of the light In some embodiments, the filter can be a hydrophilic filter or a hypophilic filter, preferably a filter suitable for reversibly attaching of the antigen.

For a filter such as used herein, it is possible to choose from a variety of materials and topologies in the prior art. Such filter can be penetrated by the solvent. The matrix should also be a suitable material for reversible capture of the scavenged antigen. In one embodiment, the method comprises contacting one or more filters to an aqueous solvent comprising antigen-binding protein molecules (antibody) bound to nanoparticles. In one embodiment, contacting said filters to the solvent leads to a release of antigens captured by said filters into said solvent.

In one embodiment, the filter is a hydrophilic filter. A hydrophilic filter is advantageous for a complete soaking of the filter with the solvent.

For example, the filter may be of the same material as FFP2 or FFP3 face masks known in the prior art. The filter has excellent properties to reliably retain an antigen. A human subject blows into the first side of the first container, said container providing a hole at the first side and tightly connected to the filter material at the second side. In one embodiment, the hole at the first side of the first container is adapted for a human subject to cover and enclose said opening with its mouth and blow exhaled air into it. In one embodiment, the second side of the first container is tightly connected to said filter. The exhaled air impinges on the filter and the filter receives dry antigens and aerosols, comprising antigens. In a significant advantage, the filter receives and captures the aerosol antigens and dry antigens from the subject's exhaled air at a rate of at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 100%. In one embodiment, the capture of the antigens by the filter is reversible. The first container is introduced with the second side, connected to the filter, into the second container the solvent. The insertion causes the filter to be completely covered by the solvent. Upon direct contact of the filter with the solvent, the captured antigens are released at a rate of at least 50%, 60%, 70% 80%, 81%, 82%, 83%, 84%, 85%, 86%, 87%, 88%, 89%, 90%, 91%, 92%, 93%, 94%, 95%, 96%, 97%, 98%, 99%, or 100% and dissolved in the solvent. In one embodiment, the filter is completely covered by the solvent for releasing antigens into the solvent. Releasing antigens into the solvents comprise pressing, mixing, flushing, and/or up and down movement of the filter in the solvent. Releasing antigens into the solvent also means dispensing or intermingling. In one embodiment, the filter is flushed in the solvent. In one embodiment, the filter can be rinsed or flushed by moving the first container up and down within the second container. In one embodiment, the filter is pressed into the solvent. In one embodiment, antigens are released and dispensed into the solvent by flushing the filter in the solvent. In one embodiment, antigens are released and dispensed into the solvent by pressing the filter in the solvent. In one embodiment, antigens are released and dispensed into the solvent by up and down movement of the filter in the solvent. This is particularly advantageous for completely or nearly completely releasing and dissolving the antigens retained on the filter into the solvent.

In one embodiment, the filter is a fibrous filter. In one embodiment, material type for the fibrous filter can be cellulose, polymers, mineral wool, regenerated cellulose, polyamides, zein fibers, cellulose acetate, cot-ten, silk, wool, carbon, activated carbon, clays, synthetic resins and cellulose derivatives, ethyl cellulose, polyamides, and/or cellulose acetate-propionate. In one embodiment, fibrous filter comprise one or more layers of one type of material described herein. In one embodiment, fibrous filter comprise two or more layers of two or more types of material.

In one embodiment, matrix of the filter has a thickness of 0.5mm, 1mm, 2mm, 3mm, 4mm, 5mm or more. In one embodiment, the maximum diameter of the matrix channels is suitable for retention an antigen used for detecting a specific pathogen. In one embodiment, the target particle size for retention by the filter can be 10 µmor less, 5 µm or less, or 2 µm or less. In one embodiment, the target particle size for retention by the filter can be 0.2 µm or less or 0.1 µm or less. In one embodiment, the pore diameter can be in the range of 0.01µm to 200µm. In one embodiment, the pore diameter can be in the range of 0.02µm to 100µm. In one embodiment, the pore diameter can be in the range of 0.02 µm to 50 µm. In one embodiment, the pore diameter can be in the range of 0.05µm to 20µm. In one embodiment, the pore diameter can be in the range of 0.075 µm to 1 µm. In one embodiment, the pore diameter can be in the range of 0.1µm to 0.75 µm. In one embodiment, the pore diameter can be in the range of 0.1µm to 0.3 µm.

In one embodiment, the filter comprise a filter with a pore diameter of at least 0.1µm and a filter thickness of less than 0.5 mm.

In one embodiment, the filter comprise a filter with a pore diameter of at least 0.1µm and a filter thickness of less than 1 mm.

In one embodiment, the filter comprise a filter with a pore diameter of at least 0.5µm and a filter thickness of less than 0.5 mm.

In one embodiment, the filter comprise a filter with a pore diameter of at least 0.5µm and a filter thickness of less than 1 mm.

In one embodiment, the filter material is FFP2 filter material. In one embodiment, the filter material is FFP3 filter material.

In one embodiment, the filter is an inertial impaction filter, with one or several nozzles and an impactor plate.

A combination, as disclosed herein, of the filter, the first container, the second container, and the solvent is surprisingly effective at capturing antigens from exhaled air, both dry and comprising aerosols, and releasing them back into the solvent. The uptake of the antigens by the filter is preferably complete or nearly complete. The release of the antigens from the filter and the dissolution of the antigens in the solvent preferably occur completely or nearly completely. This has a positive effect on accuracy, reproducibility and specificity of the measurement. The accuracy, speed, reproducibility and specificity of the measurement is particularly advantageous with this combination.

In some embodiments, the exhaled air of the human subject can be collected by direct contact of the mouth of said subject with the first container.

An advantage over the prior art is the independent and non-invasive introduction of air into the system by the human subject. This is particularly gentle on the human subject. In addition, the antigens measured are delivered directly from the human subject without further sample processing, which can lead to loss of antigens, cause contamination of the sample and delay the measurement. This can bias the result and give either a false positive or false negative measurement result. The prior art measurement methods are also uncomfortable for the human subject and may cause injury. The system of the present invention offers the significant advantage of an injury-free measurement with a highly specific and highly accurate measurement result, since fully the antigens emitted by the human subject are detected and evaluated by the system.

In some embodiments, the solvent additionally comprises a salt, a detergent, an anti-foaming agent, a stabilizing agent, a blocking agent, and/or water.

Various additives, concentrations and combinations thereof, suitable for the solvent are described herein as examples and are not intended to be limiting thereto. The prior art and one skilled in the art are aware of a variety of possible compositions for such solvents suitable to provide optimal conditions for antibody-antigen recognition, binding and color change. One skilled in the art can certainly select from the solvents in the prior art the appropriate additives, combinations and concentrations suitable for the system and method of the disclosed invention.

In some embodiments, the solvent comprises one or more of maltodextrin, trehalose, PEG, a blocking agent (e.g., BSA), and/or sodium chloride. In an exemplary embodiment, one or more of the solution components, e.g., maltodextrin, is a lyophilized bead or pellet that is suspended upon addition of a liquid, e.g., water, saline, or a liquid biological sample. It may be provided as. For example, one or more of the solution components may be provided as beads in a spectrophotometric cuvette or a reaction chamber of an analyzer rotor that are suspended in the solution upon addition of the liquid.

In one embodiment, the solvent comprises a salt. In some embodiments, the salt, NaCl, MgCI2, CaCI2, NaSCN, KCI, sodium phosphate, Tris-HCI. In one embodiment, the solvent comprises EDTA or EGTA. Salts are usually used in form of a buffer in solvents. For example, solvents comprise the buffer Phosphate-buffered saline (PBS) or Tris(hydroxymethyl)aminomethan (TRIS; e.g., TRIS-HCI) or Imidazole (e.g., Imidazole-HCI) are used. In one embodiment, the solvent comprises a detergent, such as SDS, Tween20 or TritonX-100. In one embodiment, 1% SDS or less, 0.5% SDS, 0.2% SDS or less is used. In one embodiment, 1% N-lauroylsarcosine or less, 0.5% N-lauroylsarcosine, 0.2% N-lauroylsarcosine or less is used. In one embodiment, 1% Tween20 or less, 0.5% Tween20, 0.2% Tween20 or less is used.

In one embodiment, the solvent comprises an anti-foaming agent, such as oil or waxes.

In one embodiment, the solvent comprises a stabilizing agent, such as BSA or citric acid or sodium azide, or glycine, preferably glycine and/or citric acid.

In embodiment, pH of the solvent ranges between pH 6.5 and pH 7.5, preferably between pH 6.7 and pH 7.3, more preferably close to pH 7.

In one embodiment, the solvent comprises 20 mM Tris/HCL, 150 mM sodium chlorid, 0.1% BSA, and/or Tween20, wherein Tween20 is preferably used at concentration between 0.2% and 0.4%, and wherein said solvent is preferably for detecting coronavirus antigens.

In one embodiment, the solvent comprises 20 mM PBS, 150 mM sodium chlorid, 0.1% BSA, and/or Tween20, wherein Tween20 is preferably used at concentration between 0.2% and 0.4%, and wherein said solvent is preferably for detecting coronavirus antigens.

In one embodiment, the solvent comprises 20 mM Tris/HCL, 150 mM sodium chlorid, 0.2% NP40, and/or 0.1% BSA, and wherein said solvent is preferably for detecting coronavirus antigens.

In one embodiment, the solvent comprises 20 mM PBS, 150 mM sodium chlorid, Tween20, and/or 0.1% BSA, and wherein said solvent is preferably for detecting coronavirus antigens.

The solvent is particularly advantageous here for the stability of the bond between nanoparticle and antibody. Furthermore, the antibody in the solvent has optimal molecular structures suitable for specific recognition of the antigen, wherein the antigen is completely or almost completely dissolved in the solvent. The antigen desired to be detected may be particularly well dissolved in the solvent and has an optimal molecular structure to be recognized and bound by the antibody. The binding of the antibody to the antigen results in aggregations. The binding and the aggregations lead to a change in the wavelength range of the emitted light of the nanoparticle on the antibody. The excitation light and the emitted light can pass through the solvent without interference or loss. Surprisingly, the composition of the solvent does not cause any loss of the emitted light radiation by the laser and by the nanoparticles.

In some embodiments the antibody is suitable to contact the antigen, wherein the antigen can be a protein, a peptide, carbohydrates, a lipid or a nucleic acid, preferably a peptide or a protein, more preferably a protein, wherein the antigen is an antigen of a human pathogen, such as a viral antigen, fungal antigen or a bacterial antigen, causing infectious disease, such as COVID-19, respiratory syndrome, pertussis, pneumonia, or tuberculosis.

In one embodiment, the antigen may be a nucleic acids, preferably nucleic acid of a pathogen. In one embodiment, the antigen may be a toxin, preferably a toxin of a pathogen. In one embodiment, antigen may be from a blood borne pathogen or bacteria or viruses or the like, wherein bacteria and viruses are preferably pathogens.

The antigen can be a protein, a peptide, carbohydrates, a lipid or a nucleic acid, preferably from a pathogen.

The invention described herein provides means and methods for detecting soluble antigens, wherein said antigen is preferably an antigen of a pathogen. In some embodiments, the antigens are aerosolized. In some embodiments, the antigens are solved in liquids. In some embodiments, the antigens are dry. For detecting a pathogenic bacteria or a pathogenic fungi, the antigen is preferably expressed on the surface of pathogenic bacteria or a pathogenic fungi or secreted by pathogenic bacteria or a pathogenic fungi.

In some embodiments, the system disclosed herein detect an antigen of pathogenic bacteria or a pathogenic fungi or pathogenic virus, preferably a pathogenic virus.

In some embodiments, the system disclosed herein detect an antigen of pathogene causing an infectious disease, preferably a disease of the respiratory tract, more preferably COVID-19, respiratory syndrome, pertussis, pneumonia, and tuberculosis.

In some embodiments, the system disclosed herein detect an antigen of a corona virus, an influenza virus, an upper respiratory infection virus, a pneumonia virus, a respiratory syncytial virus, a parainfluenza viruses, a metapneumovirus, a rhinovirus, preferably a coronavirus.

In some embodiments, the human pathogen is selected from a group of bacteria, fungi or viruses, such as a corona virus, influenza virus, mycobacteriaceae, streptococcae, preferably SARS corona virus, respiratory syndrome virus, streptococcus mutans, mycobacterium tuberculosis, streptococcus pneumoniae, more preferably SARS-CoV-2.

In one embodiment, the system disclosed herein detect an antigen of influenza virus, respiratory syncytial virus, parainfluenza viruses, metapneumovirus, rhinovirus, coronaviruses, adenoviruses, and/or bocaviruses, preferably coronaviruses, more preferably SARS coronavirus, even more preferably SARS-CoV-2.

In one embodiment, the system disclosed herein detect an antigen of Streptococcus pneumoniae, Streptococcus pyogenes, Haemophilus influenzae and/or Moraxella catarrhalis. In one embodiment, the system disclosed herein detect an antigen of B. anthracis spores, Bacillus anthracis vegetative and/or Vibrio cholera.

.In one embodiment, the system disclosed herein detects an antigen of Aspergillus fumigatus, Cryptococcus neoformans, Cryptococcus gattii, endemic funigi, Histoplasma Capsulatum, and/or Pneumocystis jirovecii.

In some embodiments, the viral antigen can be selected from, but not limited to, SARS-CoV-2 S-protein, SARS-CoV-2 N-protein, hPMV G protein, hPMV F protein, RSV F protein, RSV G protein, Influenza A/B protein, such as HA, MI, M2 and/or NA.

The viral antigen is recognized and bound by the antibody linked to the nanoparticle wherein the solvent comprises said antibody linked to the nanoparticle.

In one embodiment, the viral antigen is SARS-CoV-2 S-protein. In one embodiment, the viral antigen is SARS-CoV-2 N-protein.

The system and the method disclosed herein provide also means to identify the presence of influenza A and/or B by detecting the viral nucleoprotein antigenslnfluenza virus proteins suitable for detection by the system disclosed herein, include, for example, expressed proteins in the virus structure, such as HA, NA, protein polymerases (PBI, PB2, PA), matrix proteins (MI, M2), and nucleoprotein ("NP").. Immunodominant antigens of influenza comprise hemagglutinin (H) and neuraminidase (N). In influenza A, 15 subtypes of hemagglutinin (H1 - H15) and 9 subtypes of neuraminidase (N1- N9) are currently known. In one embodiment, the viral antigen of influenza can be HA, MI, M2 and/or NA antigen.

Human metapneumovirus (hPMV) and respiratory syncytial virus (RSV) are the most important cause of respiratory infections during infancy and early childhood. Both viruses code for similar surface proteins that are defined as the surface glycoprotein (G) protein and the fusion (F) protein. In one embodiment, the viral antigen is an F protein or a G protein of a hPMV. In one embodiment, the viral antigen is an F protein or a G protein of a RSV.

In some embodiments, the bacterial antigen comprises antigens including, but not limited to, pertussis-toxin, streptococcus pneumoniae C-polysaccharide, early secreted antigenic target, or culture filtrate protein.

Pathogenic bacteria may also cause a respiratory infection. In one embodiment, the system disclosed herein detect an antigen of respiratory infection causing pathogenic bacteria, such as Streptococcus pneumoniae, Streptococcus pyogenes, Haemophilus influenzae and/or Moraxella catarrhalis. In one embodiment, the bacterial antigen can be C polysaccharide of Streptococcus pneumonia. In one embodiment, the bacterial antigen can be streptolysin S, streptolysin O, toxine, exotoxine A, exotoxine C, and/or surface protein M of Streptococcus pyogenes.

As described herein, antibodies used, also recognize and bind to antigens of Mycotoxins, particularly trichothecene (T2) mycotoxins, Diacetoxyscirpenol Diverse group, Saxitoxin, or other dinoflagellage products, and/or Microcystins (various types).

The invention further relates to a method using the system disclosed herein, for antigen detection in exhaled air of a human subject comprising aerosols, wherein the antigen detection comprises:
(a) Collecting antigens from aerosols of said human subject into the system disclosed herein, by direct contact of the mouth of said subject to the first end of a first container and said container comprising a filter at the second end, and
(b) Attaching said antigens to the filter, wherein said attaching is reversible, and
(c) Dispensing said antigen into the solvent described herein by the filter being completely covered by the solvent, and
(d) Contacting said antigen with the antibody, wherein the contact causes a change of optical properties of the solvent; and is preferably a spectral sensor, and
(e) Determining said change of optical properties by means of the light sensor described herein, and transferring it to computing unit described herein, wherein said change of optical properties, and
(f) Calculating concentration of an antigen based on the change of optical properties of the solvent, wherein said concentration is a first parameter, and
wherein, said antigen detection comprises identifying at least one type of the human pathogen described herein.

The present invention, using "aerosols from exhaled air" and other non-invasively collected samples, provides a simple, non-invasive method for performing antigen detection, preferably SARS-CoV2 detection. "Non-invasive", as used herein, generally refers to a device or technique that requires minimal or no insertion into an opening, and that causes no significant discomfort to the patient. Non-invasive collection of respiratory specimens refers to collection methods that do not involve inserting an object into the patient's nose, mouth, or throat. Non-invasive collection methods can usually be performed by an individual without special medical training, but minimal assistance may be required for young children and infants. In one embodiment, the method disclosed herein comprise non-invasive sampling.

In one embodiment, the solvent comprises a mixture of two or more different antibodies, wherein each different antibody is bound to a nanoparticle comprising a specific wave range of extinction and emission. In one embodiment, the solvent comprises a mixture of two or more different antibodies detecting 1 or 2 or 3 or 4 or 5 or 6 or 7 or 8 different antigens, wherein said antigens are preferably from different pathogens. The system described herein, allow detection of more than one different antigen, also referred to as "multiplex".

In some embodiments, the present disclosure provides multiplexed assays that allow separate detection, e.g., through multiple nanoparticles of different wave spectra, with a light sensor comprising 1, 2, 3, 4, 5, 6, 7 or 8 channels that allows for multiplexed analysis.

In some embodiments, the first parameter is compared to a second parameter by the computing unit, and wherein the alarm device according to any one of claims 1-12 produces a visual and/or an acoustic signal when the first parameter is greater than or identical to the second parameter, wherein the second parameter is a pre-set value.

The preset value is a second parameter used for comparison with a first parameter. In terms of the present invention, this change includes a change in optical properties. The preset value is associated with the minimum value for detecting a change in optical properties, such as light emission, that must be achieved to define a detection as a positive detection in the sense of a detected antigen. This comparison is a simple and fast mathematical check to informatically decide whether the visual and/or audible signal is emitted, and said check can be performed by a simple computing unit.

In one embodiment, a visual and/or an acoustic signal is produced when the first parameter is identical to or greater than the second parameter.

In some embodiments, the antigen detection comprises identifying a human pathogen, preferably a virus, a fungi, or a bacteria, , more preferably corona virus, streptococcaceae, bordetella, mycobacteriaceae or influenza viruses, even more preferably SARS-CoV-2.

In some embodiments, the pathogen causes an infectious disease in a human subject. Pathogens include microorganisms, viruses, and toxins that can cause disease. The antigen is not required to be infectious itself, but may indicate, i.e. used for detection of, the presence of a pathogen.

For detecting a pathogenic bacteria or a pathogenic fungi, the antigen is preferably expressed on the surface of pathogenic bacteria or a pathogenic fungi or secreted by pathogenic bacteria or a pathogenic fungi.

In one embodiment, the system disclosed herein detect an antigen of influenza virus, respiratory syncytial virus, parainfluenza viruses, metapneumovirus, rhinovirus, coronaviruses, adenoviruses, and/or bocaviruses, preferably coronaviruses, more preferably SARS coronavirus, even more preferably SARS-CoV-2.

In the sense of the invention, antigens of Coronaviridae are preferably detected. In one embodiment, antigen detection comprises identifying a human pathogenic coronaviruses, including human coronavirus OC43 (HCoV-OC43), human coronavirus HKU1 (HCoV-HKU1), human coronavirus 229E (HCoV-229E), human coronavirus NL63 (HCoV-NL63), Middle East respiratory syndrome-related coronavirus (MERS-CoV), severe acute respiratory syndrome coronavirus (SARS-CoV), and severe acute respiratory syndrome coronavirus 2 (SARS-CoV-2), preferably SARS-CoV-2.

A particular advantage of the present system is that more than one antigen can be detected and that this detection of more than one antigen can also occur simultaneously, also known as multiplex. Furthermore, it is advantageous that the antigens can be assigned to a specific pathogen. This is particularly beneficial for diagnostics, therapy and classification of a risk to the infected human subject and the human subjects in the proximity of the infected human subject. The proximity is suitable to enable a transmission of the pathogen from the infected human subject to another human subject.

Surprisingly, a multiplex measurement is as fast, sensitive and specific as the single detection. The skilled person could not expect that the color detection of the nanoparticles do not interfere with each other, e.g. by scattering, light energy depletion, resonance energy transfer. Further unexpectedly, the sensitivity or specificity of antigen-antibody binding is unchanged in multiplex compared to single detection.

Further examples of system and methods are described in detail below.

### DETAILED DESCRIPTION OF THE INVENTION

The present invention relates to a system for antigen detection comprising a first container configured to collect aerosols from exhaled air of a human subject and a second container suitable for a solvent comprising nanoparticles linked to antibodies. In a further aspect of the invention, a change of optical properties of the solvent is detectable upon contact between the antibodies and matching antigens and wherein said change is associated with the presence of an antigen.

Unless otherwise explained, all technical and scientific terms used herein have the same meaning as commonly understood by one person skilled in the art to which the disclosure belongs. The singular terms "a," "an," and "the" include plural referents unless context clearly indicates otherwise. "Comprising" means "including." Hence "comprising A or B" means "including A" or "including B" or "including A and B."

### Aerosols

The term "aerosol" as used herein also includes a droplet, a droplet, and a droplet core. An aerosol, as used in the invention, is understood as a heterogeneous mixture of solid and/or liquid components produced by a human subject and comprised in the exhaled air of said human subject.

### Aerosol particles, exhaled breath

The term "aerosol particle," as used herein, is also understood to include a component of a pathogen or the pathogen exhaled from a human subject. The aerosol particle is also the solid component in an aerosol. For purposes of the invention, the aerosol particle travels in exhaled air bound to a liquid component, the aerosol, and/or as an aerosol particle in a crystal structure. The diameter of aerosol particles is between preferably 0.01 µm to 500 µm, more preferably 0.1 µm to 100 µm, most preferably 0.1 µm to 50 µm.

### First Container

For purposes of the invention, the first container refers to a vessel that has a cavity within it. In preferred embodiments, the first container may have a substantially cylindrical, ellipsoidal or spherical shape. Preferably, the first container is configured to allow a user to exhale, particularly blow, air into the first container. For example, a user may pick up the first container itself and exhale air therein. Also preferably, the first container may be guided to the user of the preferred system by another person. In further embodiments, the first container may be attached to a holder during use.

### First side and second side of first container

In preferred embodiments, the first container includes a first side and a second side. Preferably, the first side and the second side designate regions of the first container. Preferably, the first side and the second side may describe extremities of the first container to some extent. For example, in the preferred case of a cylindrical embodiment of the first container, the first side and the second side may form the bottom and the lid. Preferably, the first side of the container includes a hole and the second side includes a filter.

### Hole

For purposes of the invention, the hole preferably refers to an open area of the first container, preferably located on the first side of the first container. The hole preferably allows exhaled air from a user to enter the cavity of the first container. The hole of the first container may preferably extend substantially along the first side of the container. In further preferred embodiments, the hole may extend along a portion of the first side. In further embodiments, a movable body is located within the cavity of the first container such that a substantially regularly recurring cross-sectional change in the hole occurs as a result of exhaled air, such that a user receives acoustic feedback upon exhalation (similar to a whistle), which may have an effect such as a pitch change.

### Second container

Preferably, the second container refers to a vessel that has a cavity in its interior. In preferred embodiments, the second container may have a substantially cylindrical, ellipsoidal or spherical shape. Preferably, the container is configured to have the solvent placed within the second container. In addition, it is preferred that the first container is adapted to fit substantially snugly within the cavity of the second container. It is likewise preferred that there is substantially airtight contact between the first container and the second container. To this end, it is preferred that the geometric configuration of the first container and the second container is substantially identical. Preferably, the diameter of the second container is slightly larger than that of the first container and/or is substantially the same as that of the cavity of the first container. In further preferred embodiments, the second container may be a component or part of a support frame that includes preferred components of the system of the invention. Thus, when the first container is fed into the second container, aerosols that are preferably in the filter are contacted with the solvent of the first container. Preferably, the second container is replaceable. Furthermore, it is preferred that the second container has a shell surface comprising substantially transparent material so that light can strike the nanoparticles located in the solvent starting from the light source.

### Measuring unit

In the context of the invention, the measuring unit preferably denotes a spatial region in which the preferred measurement of optical properties of the solvent, in particular a change in optical properties of the solvent after the first container has been fed into the second container. In preferred embodiments, the measuring unit may be located within a carrier frame. Thus, the measuring unit preferably designates in volume in which the light source irradiates the solvent and optical properties can be detected by the light sensor, preferably the corresponding components of the preferred system can be mounted in the measuring unit accordingly.

### Spectrometer

The design and operation of spectrometers are well known in the prior art. In the sense of the invention, the spectrometer denotes an arrangement comprising the light source and the light sensor. Thereby, it is preferred that the second container is placed between the light source and the light sensor in order to be able to perform a measurement of a change in optical properties by the light sensor. Furthermore, it is preferred that further optical components, such as lenses, mirrors, prisms and/or gratings, are positioned in a beam path between the light source and the light sensor, for example, in order to adapt and/or adjust the beam path. In particular, divergent beam paths can be advantageously compensated by further optical components.

### Sealing

In the sense of the invention, a sealing preferably denotes an element and/or a construction which preferably avoids an undesired transition of the solvent into the first container, but preferably enables a desired transition simultaneously, namely by introducing the first container into the second container through the filter the first filter enters the first container. In this regard, the sealing may be, for example, a translational seal, such as, but not limited to, a stuffing box, a piston ring, a bellows, a brush seal, a hydraulic seal, a pneumatic seal, and/or a carbon fin

### Shuttling stroke motion

The phrase shuttling stroke motion preferably refers to a lifting motion of the first container into the second container. Here, in the context of the invention, a stroke motion comprises a substantially vertical up and down movement of the first container into the second container. Preferably, the lifting movement may be repetitive. The lifting movement, in particular the repeated lifting movement of the first container, can flush aerosols that were previously attached to the filter into the solvent.

### Light source, Light sensor

In a further embodiment, the system further comprises exposing the conjugate to a light source in a wavelength range within the ultraviolet-visible-infrared spectrum. In yet a further aspect, the method comprises measuring a light signal from the complex, wherein a change in the light signal is indicative of the presence of the target analyte in the sample.

In another embodiment, the system comprises exposing the conjugate to a light source in the wavelength range within the ultraviolet, visible, and infrared spectra; and measuring a light signal from the conjugate, wherein the change in the light signal indicates the presence of the target analyte in the sample.

### Alarm device

In the context of the invention, an alarm device is a device that is capable of triggering an alarm signal if variable optical properties of the solvent are detected. The alarm signal can be, for example, an acoustic or optical signal.

### Optical properties

The optical properties preferably refer to those properties and/or effects that appear when light passes through the solvent starting from the light source. In particular, a change in optical properties occurs when antigen matching the antibody on the nanoparticles is present on the aerosol, so that the nanoparticles agglomerate on the antigen. This also exerts an effect on the irradiated light. In particular, the scattering properties of the light change. In particular, the optical properties affect the scattering. The phenomenon that part of the light is deflected from its rectilinear path is scattering. However, other effects such as, for example, reflection and absorption, can also be relevant in the context of the invention.

### Computing unit

The term "computing unit" preferably refers to any device that can be configured to perform computing operations. Preferably, the computing unit is a processor, a processor chip, a microprocessor, and/or a microcontroller that is preferably configured to perform an evaluation of the measured change in optical properties. The computing unit may also preferably be a programmable circuit board to process a change in optical properties. The computing unit may also preferably comprise a computer-usable or computer-readable medium, such as a hard disk, random access memory (RAM), read-only memory (ROM), flash memory, etc.

The phrase "computing unit configured to do so" preferably means that, in order to perform certain computing operations, such as determining the type and/or amount of antigens by changing the optical properties, computer code and/or software is installed on the computing unit, preferably capable of performing such computing operations. The computer code and/or software may be written in any programming language or model-based development environment, such as C/C++, C#, Objective-C, Java, Basic/VisualBasic, MATLAB, Simulink, StateFlow, Lab View, or Assembler.

The computer code and/or software, which is preferably installed on the computing unit, can be considered as technical features since a direct physical effect of the system, for example by irradiating the solvent by means of the light source, is used. Functional descriptions of the computer code and/or software may therefore be considered as preferred and defining embodiments of the invention. The particular computer code and/or software used is available to those skilled in the art and can be installed accordingly using standard skill in the art.

In preferred embodiments, reference data may also be available stored on the computer unit, which preferably allows correlation of the change in optical properties for the measurement process,

Reference data can thus relate to data that enable a correlation of the change in the optical properties with the antigens.

The reference data may be provided, for example, as a look-up table or as parameters for a mathematical relationship that enables correlation of the physical quantities. For example, the reference data may be obtained by performing appropriate calibration experiments that measure the relationship between the change in optical properties and amount and/or type of antigens.

Typically, the reference data can be stored on a computer-usable or computer-readable medium in the control unit. Any format commonly used in the industry may be used. The reference data may be stored in a separate file and/or integrated into the computer code or software (e.g., source code) that is present on the computing unit.

### Monochromatic laser

For the purposes of the invention, a monochromatic laser preferably refers to a laser that emits monochromatic, i.e., single-color, and thus light of substantially one wavelength or frequency.

*Pathogen-nanoparticle agglomerates.*

A pathogen-nanoparticle agglomerates preferably refers to a substantially solid accumulation of multiple nanoparticles, which preferably appears when antibodies thereon couple with matching antigens. As a result, multiple nanoparticles are agglomerated into a cluster, which alters optical properties of the solvent when irradiated with the light source and can be measured.

### Extinction peak wavelength

The term extinction peak wavelength preferably refers to the optical wavelength at which the maximum light intensity gets absorbed and/or scattered.

### Spectral sensor

A spectral sensor preferably refers to a sensor that is set up to split the incident light according to wavelength with the aid of one or more detector components, so that the entire spectral distribution of the light can preferably be detected simultaneously. In particular, the spectral sensor can detect the light of the scattered light. Spectral sensors thus allow detailed access to the spectral information in the radiation flux.

### Channels of spectral sensor

Channels of spectral sensor preferably denotes measurement channels of the spectral sensor, which are preferably assigned to detector components in order to detect light of different ranges of a wavelength or a frequency.

### Raman shifted light scattered

In the sense of the invention, Raman shifted light scattered preferably means light that is scattered by the effect of Raman scattering and is detectable by the light sensor.

In order to detect the light scattered by Raman scattering, the polarizability must change upon rotation or vibration of the molecule. In the spectrum of the light scattered by the sample on the solvent by agglomeration of the nanoparticles (in the case of antigens matching the antibodies), other frequencies are observed in addition to the irradiated frequency (Rayleigh scattering). The frequency differences to the irradiated light correspond to the energies of rotation, oscillation, phonon or spin-flip processes characteristic for the material. The reason lies in an interaction of light with matter, the so-called Raman effect, in which energy is transferred from light to matter ("Stokes side" of the spectrum), or energy is transferred from matter to light ("anti-Stokes side" of the spectrum). Since the wavelength of light, i.e., its color, depends on the energy of the light, this energy transfer causes a shift in the wavelength of the scattered light relative to the incident light, called the Raman shift. Raman scattering typically has a small scattering cross section, which is relevant for increasing the sensitivity of the preferred system.

### In line

The phrase "in line" preferably refers to such an arrangement of the light source and the light sensor, preferably the spectral sensor, that the light source and the light sensor form a horizontal line as a connecting line. By an in line arrangement of the light source and the light sensor a particularly precise detection of a change of optical properties can be made possible.

### Mie scattered light

Mie scattered light is the preferred term for light resulting from the Tyndall effect. In Mie scattering, light is scattered by nanoparticles with a diameter similar to or larger than the wavelength of the incident light. The Mie scattering signal is proportional to the square of the particle diameter of the scattering particle.

### Linear polarized filter

A linear polarized filter preferably refers to a component that can be used to polarize unpolarized light into linearly polarized light. This allows light of only one polarization direction to pass through.

### Depolarized light

In the sense of the invention, preferably depolarized light means light which oscillates in any direction perpendicular to the direction of propagation.

### Visual signal

A visual signal preferably means a signal that can be visually perceived by a human being, for example by a display, a light signal, starting from a flashing lamp, an LED; a color change, etc.

### Acoustic signal

An acoustic signal preferably refers to a sound wave that lies in the frequency range of approximately 10 to 20,000 Hz and is thus perceptible to the human ear.

### Communication unit

In the sense of the invention, a communication unit preferably means a transmitting unit for transmitting a signal which preferably sends information about the change in optical properties and/or about antigens. The transmission of data and/or information via the communication unit is preferably carried out by means of directed or non-directed electromagnetic waves, whereby the range of the frequency band used can vary from a few hertz (low frequency) to several hundred terahertz (visible light), depending on the application and the technology used. According to the invention, a wide variety of data transmission methods can be used, with Bluetooth, WLAN, ZigBee, Thread, RFID, NFC, Wibree or cellular mobile radio networks (especially campus networks) or WiMAX in the radio frequency range as well as IrDA and optical directional radio (FSO) in the infrared or optical frequency range being mentioned by way of example. Preferably, information can be transmitted to an external data processing unit by the communication unit.

In the sense of the invention, an external data processing unit preferably refers to an external computing unit that can be set up to read out data, preferably also to evaluate data. For example, the external data processing unit may be a PC, a tablet computer and/or a mobile terminal such as a smartphone.

### Spreading

The term " spreading " includes diffusion, transport and convection. In particular, dispersion may occur via air currents and air flow. In particular, for the purposes of the invention, the term means the spread of viruses, pathogens, aerosol particles, aerosols, salt crystals and/or salts dissolved in aerosols. It can be understood by spreading the virus spreading, aerosol particle spreading, aerosol spreading, crystal spreading, pathogen spreading. Diffusion is the equalization of concentration differences in liquids or gases due to Brownian molecular motion. Convection is the transport of physical quantities in flowing gases or liquids.

### Direct spatial contact

For the purposes of the invention, the term "direct spatial contact" preferably means that two objects are in contact.

### Continuous/ periodic/ one-time

The term "periodic", as used here, describes in particular a process that occurs regularly and recurrently at equal intervals over a defined period of time. "Continuous" is also understood to mean a process occurring continuously over a defined period of time. For the purposes of the invention, an operation is also referred to as "one-time" if it is triggered once and can be triggered repeatedly at unequal intervals over an undefined period of time.

### Medical Indications

In one aspect of the present invention there is provided a system and a method comprising an antibody or antibody fragment bound to a nanoparticle according to the invention as herein described for use in the detection of an pathogen of a medical condition associated with a SARS Coronavirus, wherein the medical condition associated with a SARS Coronavirus is preferably COVID-19 or a SARS Coronavirus-associated respiratory disease.

As used herein, the "patient" or "subject" may be a vertebrate. In the context of the present invention, the term "subject" includes both humans and animals, particularly mammals, and other organisms.

As used herein, a patient with "symptoms of a viral infection of the respiratory tract" is a subject, who presents with one or more of, without limitation, cold-like symptoms or flu-like illnesses, such as fever, cough, runny nose, sneezing, sore throat, having trouble breathing, headache, muscle aches, fatigue, rapid pulse, rapid breathing, nausea and vomiting, lack of taste and/or smell and/or malaise (feeling unwell).

In some embodiments, symptoms of infection with a SARS-virus are fever, sore throat, cough, myalgia or fatigue, and in some embodiments, additionally, sputum production, headache, hemoptysis and/or diarrhea. In some embodiments, symptoms of an infection with a SARS-coronavirus, for example SARS-CoV-2, are fever, sore throat, cough, lack of taste and/or smell, shortness of breath and/or fatigue.

As used herein, the term "a patient that is at risk of developing a severe acute respiratory syndrome (SARS)" relates to a subject, preferably distinct from any given person in the general population, who has an increased (e.g. above-average) risk of developing SARS. In some embodiments, the patient has symptoms of SARS or symptoms of a SARS Coronavirus infection. In some embodiments, the patient has no symptoms of SARS or symptoms of a SARS Coronavirus infection. In some embodiments, the subject has been in contact with people with SARS Coronavirus infections or symptoms. In some embodiments, the person at risk of developing SARS has been tested for the presence of a SARS Coronavirus infection. In some embodiments, the person at risk of developing SARS has tested positive for the presence of a SARS Coronavirus infection, preferably a coronavirus infection.

In embodiments, the patient at risk of developing SARS is an asymptomatic patient that shows no specific symptoms of SARS (yet). An asymptomatic patient may be at risk of developing SARS because the patient has been in contact with a person infected with a SARS Coronavirus. For example, the asymptomatic patient may have been identified as being at risk of developing SARS by a software application (app) that is installed on his smart phone or corresponding (portable) device and that indicates physical proximity or short physical distance to an infected patient that uses a corresponding app on its respective mobile device/smart phone. Other methods of determining contact/physical proximity to an infected person are known to the skilled person and equally apply to the method of the invention.

In some embodiments, the patient that has or is at risk of developing a severe acute respiratory syndrome (SARS) has a coronavirus infection.

Coronaviruses are a group of related viruses that cause diseases in mammals and birds. The scientific name for coronavirus is Orthocoronavirinae or Coronavirinae. Coronavirus belongs to the family of Coronaviridae. The family is divided into Coronavirinae and Torovirinae sub-families, which are further divided into six genera: Alphacoronavirus, Betacoronavirus, Gammacoronavirus, Deltacoronavirus, Torovirus, and Bafinivirus. While viruses in the genera Alphacoronaviruses and Betacoronaviruses infect mostly mammals, the Gammacoronavirus infect avian species and members of the Deltacoronavirus genus have been found in both mammalian and avian hosts.

In humans, coronaviruses cause respiratory tract infections that can be mild, such as some cases of the common cold, and others that can be lethal, such as SARS, MERS, and COVID-19. Coronaviruses are enveloped viruses with a positive-sense single-stranded RNA genome and a nucleocapsid of helical symmetry. The genome size of coronaviruses ranges from approximately 27 to 34 kilobases, the largest among known RNA viruses.

Various species of human coronaviruses are known, such as, without limitation, Human coronavirus OC43 (HCoV-OC43), of the genus β-CoV, Human coronavirus HKU1 (HCoV-HKU1), of the genus β-CoV, Human coronavirus 229E (HCoV-229E), α-CoV, Human coronavirus NL63 (HCoV-NL63), α-CoV, Middle East respiratory syndrome-related coronavirus (MERS-CoV), Severe acute respiratory syndrome coronavirus (SARS-CoV), Severe acute respiratory syndrome coronavirus 2 (SARS-CoV-2).

Coronaviruses vary significantly in risk factor. Some can kill more than 30% of those infected (such as MERS-CoV), and some are relatively harmless, such as the common cold. Coronaviruses cause colds with major symptoms, such as fever, and a sore throat, e.g. from swollen adenoids, occurring primarily in the winter and early spring seasons. Coronaviruses can cause pneumonia (either direct viral pneumonia or secondary bacterial pneumonia) and bronchitis (either direct viral bronchitis or secondary bacterial bronchitis). Coronaviruses can also cause SARS.

In some embodiments, the patient suffers from an infection, preferably with a SARS Coronavirus (SARS-CoV). As used herein, SARS Coronavirus refers to a Coronavirus that leads to severe acute respiratory syndrome (SARS). This syndrome is a viral respiratory disease of zoonotic origin that first surfaced in the early 2000s caused by the first-identified strain of the SARS coronavirus (SARS-CoV or SARS-CoV-1).

SARS is induced via droplet transmission and replication of the virus could be shown in the upper and lower respiratory tract or gastrointestinal mucosa. In parallel the virus is also capable of directly invading cells of different organs such as liver, kidney, heart and brain. Another distinct mechanism appears to be the direct invasion of the virus in T-cells. A significant number of SARS patients, who suffer COVID-19, have a clinically low concentration of lymphocytes in the blood, also known as Lymphocytopenia. Clinically, patients show respiratory symptoms such as dry cough and shortness of breath, fever or diarrhea. But symptoms associated with acute liver injury, heart injury or kidney injury can also occur. In a less severe state of SARS, patients can show mild or even no symptoms.

Embodiments of a SARS Coronavirus include, without limitation, any coronavirus that induces a SARS or SARS-similar pathology. Particular embodiments include, without limitation, the SARS Coronavirus (SARS-CoV-1) first discovered in 2003 (as described above), the Middle East respiratory syndrome (MERS-CoV) first discovered in 2012, and the SARS-CoV-2, which causes COVID-19, a disease which brought about the 2019-2020 coronavirus pandemic.

The strain SARS-CoV-2 causes COVID-19, a disease which brought about the ongoing 2019-2020 coronavirus pandemic. The disease was first identified in December 2019 in Wuhan, the capital of China's Hubei province, and spread globally. Common symptoms include fever, cough, and shortness of breath. Other symptoms may include muscle pain, diarrhea, sore throat, loss of taste and/or smell, and abdominal pain. While the majority of cases result in mild symptoms, some progress to viral pneumonia and multi-organ failure.

Coronaviruses can be determined by molecular techniques, for example sequence-based analysis, for example by PCR-based amplification of viral genetic material. Genome-wide phylogenetic analysis indicates that SARS-CoV-2 shares 79.5% and 50% sequence identity to SARS-CoV and MERS-CoV, respectively. However, there is 94.6% sequence identity between the seven conserved replicase domains in ORF1ab of SARS-CoV-2 and SARS-CoV, and less than 90% sequence identity between those of SARS-CoV-2 and other -CoVs, implying that SARS-CoV-2 belongs to the lineage of Beta-CoVs.

Similar to other CoVs, the SARS-CoV-2 virion with a genome size of 29.9 kb possesses a nucleocapsid composed of genomic RNA and phosphorylated nucleocapsid (N) protein. The nucleocapsid is buried inside phospholipid bilayers and covered by two di erent types of spike proteins: the spike glycoprotein trimmer (S) that exists in all CoVs, and the hemagglutininesterase (HE) only shared among some CoVs. The membrane (M) protein and the envelope (E) protein are located among the S proteins in the viral envelope. The SARS-CoV-2 genome has 5' and 3' terminal sequences (265 nt at the 5' terminal and 229 nt at the 3' terminal region), which is typical of -CoVs, with a gene order 5'-replicase open reading frame (ORF) 1ab-S-envelope(E)-membrane(M)-N-30. The predicted S, ORF3a, E, M, and N genes of SARS-CoV-2 are 3822, 828, 228, 669, and 1260 nt in length, respectively. Similar to SARS-CoV, SARS-CoV-2 carries a predicted ORF8 gene (366 nt in length) located between the M and N ORF genes.

### Infectious diseases, viruses, pathogen, SARS-COV-2

The term "infectious disease", as used herein, is also understood in the sense of the invention to mean a disease in humans triggered by contact and/or penetration of pathogens into cells, preferably triggered by viruses. The person skilled in the art understands that "viruses" also means organic structures which, as virions, spread outside cells by transmission, but which, as viruses, can only multiply within a suitable cell. The person skilled in the art understands that the entry of viruses into cells may also be referred to as infectious or potentially infectious.

### Pathogens

A "pathogen", as used herein, refers to a molecular structure or agent possessing the ability to be infectious, i.e. to cause disease, such as an infectious disease, in certain organisms. In some embodiments, the pathogen causes an infectious disease in a human subject. Pathogens include microorganisms, viruses, and toxins that can cause disease. The antigen is not required to be infectious itself, but may indicate, i.e. used for detection of, the presence of a pathogen.

The invention described herein provides means and methods for detecting soluble antigens, wherein said antigen is preferably an antigen of a pathogen. In some embodiments, the antigens are aerosolized. In some embodiments, the antigens are solved in liquids. In some embodiments, the antigens are dry. For detecting a pathogenic bacteria or a pathogenic fungi, the antigen is preferably expressed on the surface of pathogenic bacteria or a pathogenic fungi or secreted by pathogenic bacteria or a pathogenic fungi.

Pathogenic fungi: Pulmonary fungal pathogens are able to cause life-threatening invasive diseases. Pathogenic fungi causing a respiratory disease include Aspergillus, Cryptococcus, endemic fungi, and Pneumocystis. In one embodiment, the system disclosed herein detect an antigen of Aspergillus fumigatus, Cryptococcus neoformans, Cryptococcus gattii, endemic funigi, Histoplasma Capsulatum, and/or Pneumocystis jirovecii.

As described herein, antibodies used, also recognize and bind to antigens of Mycotoxins, particularly trichothecene (T2) mycotoxins, Diacetoxyscirpenol Diverse group, Saxitoxin, or other dinoflagellage products, and/or Microcystins (various types).

Pathogenic bacteria: As described herein, antibodies used, recognize and bind to antigens of pathogenic bacteria, wherein pathogenic bacteria can be selected from a group of, but not limited to, B. anthracis spores, Bacillus anthracis vegetative, Francisella tularensis, Yersinia pestis, Vibrio cholerae 0139, Vibrio cholerae O1, Brucella spp, Chlamydia spp, Bacillus subtilis spores, Staphylococcal enterotoxin-B (SEB) and other superantigens, ricin, pertussis toxin, Salmonella spp, Shigella dysenteriae, E. coli O157:H7, Ralstonia spp, Listeria, or Clostridium botulinum. Pathogenic bacteria causing a respiratory infection include Streptococcus pneumoniae, Streptococcus pyogenes, Haemophilus influenzae and Moraxella catarrhalis. In one embodiment, the system disclosed herein detect an antigen of Streptococcus pneumoniae, Streptococcus pyogenes, Haemophilus influenzae and/or Moraxella catarrhalis.

Pathogenic viruses: As described herein, antibodies used, recognize and bind to antigens of pathogenic virus, wherein pathogenic virus can be selected from a group of, but not limited to, corona virus, influenza virus, upper respiratory infection virus, pneumonia, respiratory syncytial virus, parainfluenza viruses, metapneumovirus, rhinovirus, coronaviruses, adenoviruses, bocaviruses, Venezuelan Equine Encephalitis virus, foot-and-mouth disease virus, Dengue virus, Orthopoxviruses, Potyvirus. In one embodiment, pathogenic virus is preferably a virus causing a respiratory infection, such as pneumonia or upper respiratory infection. Pathogenic virus causing a respiratory infection include influenza virus, respiratory syncytial virus, parainfluenza viruses, metapneumovirus, rhinovirus, coronaviruses, adenoviruses, and bocaviruses. In one embodiment, the system disclosed herein detect an antigen of influenza virus, respiratory syncytial virus, parainfluenza viruses, metapneumovirus, rhinovirus, coronaviruses, adenoviruses, and/or bocaviruses, preferably coronaviruses, more preferably SARS coronavirus, even more preferably SARS-CoV-2.

Several families of human pathogenic viruses are known, as listed here, non-exhaustively: Coronaviridae, Herpesviridae, Hepadnaviridae, Flaviviridae, Retroviridae, Filoviridae, Orthomyxoviridae, Paramyxoviridae, Pneumoviridae, Rhaboviridae, Adenoviridae, Polyomaviridae, Papillomaviridae, Picornaviridae, preferably in the sense of the invention Coronaviridae. Of the human pathogenic coronaviruses , e.g., without limitation, human coronavirus OC43 (HCoV-OC43), human coronavirus HKU1 (HCoV-HKU1), human coronavirus 229E (HCoV-229E), human coronavirus NL63 (HCoV-NL63), Middle East respiratory syndrome-related coronavirus (MERS-CoV), severe acute respiratory syndrome coronavirus (SARS-CoV), and severe acute respiratory syndrome coronavirus 2 (SARS-CoV-2) are known, preferably SARS-CoV-2. In Table 1, examples for virus families comprising a pathogenic virus and a corresponding immunogen that can be used for

| **Family** | **Virus example** | **Immunogen** |
|---|---|---|
| Orthomyxovirid ae | Influenza | HA (HA2) |
| Paramyxovirida e | PIV5 SER | F (F1) |
| Retroviridae | HIV MLV | Env (gp41) |
| Coronaviridae | SARS | S (S2) |
| Arenaviridae | LCMV | GP (GP2 + SSP) |
| Filoviridae | EBOV | GP (GP2) |
| Togaviridae | SFV | E1 |
| Flaviviridae | TBE | E |
| Bunyaviridae | UUKV | Gc |
| Rhabdoviridae | VSV | G |
| Herpesviridae | HSV-1 | gB |
| Bornaviridae | BDV | GP (GP2)f |
| Poxviridae | VV | EFC |
| Asfarviridae | ASFV | |
| Arteriviridae | PRRSV | |
| Hepadnavirida e | HBVg | |

For influenza viruses, the immunodominant antigens located on the surface of the virus are hemagglutinin (H) and neuraminidase (N). In influenza A, 15 subtypes of hemagglutinin (H1 - H15) and 9 subtypes of neuraminidase (N1 - N9) are currently known. Influenza virus proteins suitable for detection by the system disclosed herein, include, for example, expressed proteins in the virus structure, such as HA, NA, protein polymerases (PBI, PB2, PA), matrix proteins (MI, M2), and nucleoprotein ("NP"). Preferably, the conserved peptide sequences are conserved on at least two or more of the MI, M2, HA, NA, or one or more polymerase proteins, and preferably a majority or even all of the preceding.

### Spike protein, corona proteins

Of particular interest in terms of diagnostics are the spike protein (S protein), due to its role in interaction with human host receptors, and the highly conserved nucleocapsid protein (N protein) with high immunogenicity.

The Coronavirus Spike protein, also known as S protein, is a glycoprotein trimer, wherein each monomer of the trimeric S protein is about 180 kDa, and contains two subunits, S1 and S2, mediating attachment and membrane fusion, respectively. As described in Xiuyuan Ou et al (Nature Communications, 11, 1620, 2020), Coronaviruses (CoV) use the Spike glycoprotein to bind ACE2 and mediate membrane fusion and virus entry. In the S protein structure, N- and C-terminal portions of S1 fold as two independent domains, N-terminal domain (NTD) and C-terminal domain (C-domain). While RBD of mouse hepatitis virus (MHV) is located at the NTD14, most of other CoVs, including SARS-CoV and MERS-CoV use C-domain to bind their receptors.

In one embodiment, the antibody bound to the nanoparticle recognizes the S protein and is used to identify SARS-CoV-2 infected human subjects in the system and method of the invention. In one embodiment, the antibody bound to the nanoparticle specifically recognizes a domain of the S-protein, such as a human recombinant monoclonal antibody or antibody fragment that recognizes the receptor binding domain (RBD) of the S1 subunit (S1) of a recombinant Spike glycoprotein of SARS-CoV-2

Another protein that can be used as antigen to detect SARS-CoV-2 in aerosols of a human subject is the SARS-CoV-2 Coronavirus Nucleocapsid.

SARS-CoV-2 nucleocapsid protein (also SARS-CoV-2 N protein or N-protein) is a highly immunogenic phosphoprotein and is the most abundantly detectable protein in coronaviruses. SARS-CoV-2 N protein is involved in viral genome replication and cell signaling pathway modulation. The SARS-CoV-2 nucleocapsid protein is of great interest for the development of diagnostic SARS-CoV-2 assays because the sequence of the N protein is highly conserved and exhibits strong immunogenicity. SARS-CoV-2 nucleocapsid protein is the major structural protein that binds the viral RNA genome. In one embodiment, the antibody bound to the nanoparticle recognizes the N protein and is used to identify SARS-CoV-2 infected human subjects in the system and method of the invention. In one embodiment, the antibody bound to the nanoparticle specifically recognizes a domain of the N-protein, such as a human recombinant monoclonal antibody, human recombinant polyclonal antibody or antibody fragment.

In some embodiments, the antibodies used may therefore be CoV-2 specific, in other words, they may bind the Spike protein or the N-protein of the CoV-2 and not of other viruses. In some embodiments, the antibodies may bind the Spike protein or the N-protein of any given SARS Coronavirus. In some embodiments, the antibodies may bind the Spike protein or the N-protein of a Coronavirus that evolves or mutates from CoV-2 to form a new virus, albeit with sufficient similarity to be bound by the antibodies. In some embodiments, the antibodies bind a SARS Coronavirus (also referred to herein as a "Coronavirus"), more preferably to SARS-CoV-2. The antibodies used are therefore considered to bind the RBD of the Spike protein or the N-protein of SARS-CoV-2 as it exists in subjects, i.e. in the form it takes in human subjects before, during or after infection of a host cell.

### Antibody, Antigen

As used herein, an "antibody" generally refers to a protein consisting of one or more polypeptides substantially encoded by immunoglobulin genes or fragments of immunoglobulin genes that binds or recognize or contacts specifically to an epitope of an antigen. Antibodies include monoclonal antibodies, polyclonal antibodies or fragments of antibodies, and other forms known in the art. In some examples, an antibody is linked or conjugated to another molecule, such as a nanoparticle (e.g., a gold nanoparticle). The terms "polypeptide," "peptide," or "protein", as used herein, refer to a polymer in which monomers are amino acid residues linked by amide bonds (e.g., an amino acid sequence) and include modified sequences such as glycoproteins. If the amino acids are alpha-amino acids, either the L-optic isomer or the D-optic isomer may be used. The term "polypeptide" or "protein" refers to both naturally occurring and recombinantly or synthetically produced proteins.

Where the term "antibody" is used, the term "antibody fragment" may also be considered to be referred to. The recognized immunoglobulin genes include the kappa, lambda, alpha, gamma, delta, epsilon and mu constant region genes, as well as the myriad immunoglobulin variable region genes.

Antibodies consist of a heavy chain and a light chain, each with a variable region called the variable heavy region (VH) and variable light region (VL). Together, the VH region and the VL region are responsible for binding the antigen recognized by the antibody. These include intact immunoglobulins and the variants and parts of them known in the art, such as Fab' fragments, F(ab)'2 fragments, single-chain Fv proteins ("scFv"), and disulfide-stabilized Fv proteins ("dsFv"). An scFv protein is a fusion protein in which an immunoglobulin light chain variable region and an immunoglobulin heavy chain variable region are joined by a linker, whereas in dsFvs the chains have been mutated to introduce a disulfide bond to stabilize the joining of the chains. The term also includes recombinant forms such as chimeric antibodies (e.g., humanized murine antibodies) and heteroconjugated antibodies (e.g., bispecific antibodies). See also: Pierce Catalog and Handbook, 1994-1995 (Pierce Chemical Co., Rockford, IL); Kuby, Immunology, 3rd Ed., W.H. Freeman & Co., New York, 1997).

Light chains are classified as either kappa or lambda. Heavy chains are classified as gamma, mu, alpha, delta, or epsilon, which in turn define the immunoglobulin classes, IgG, IgM, IgA, IgD, and IgE, respectively. The basic immunoglobulin (antibody) structural unit is known to comprise a tetramer or dimer. Each tetramer is composed of two identical pairs of polypeptide chains, each pair having one "light" (L) (about 25 kD) and one "heavy" (H) chain (about 50-70 kD). The N-terminus of each chain defines a variable region of about 100 to 110 or more amino acids, primarily responsible for antigen recognition. The terms "variable light chain" and "variable heavy chain" refer to these variable regions of the light and heavy chains respectively. Optionally, the antibody or the immunological portion of the antibody, can be chemically conjugated to, or expressed as, a fusion protein with other proteins.

A "monoclonal antibody" is an antibody produced by a single clone of B lymphocytes or by a cell into which the light and heavy chain genes of a single antibody have been transfected. Monoclonal antibodies are produced by methods known to those skilled in the art, for example, by producing hybrid antibody-forming cells from a fusion of myeloma cells with immune spleen cells. These fused cells and their progeny are referred to as "hybridomas". Monoclonal antibodies include humanized monoclonal antibodies.

The term "conjugate" or "bio-conjugate" refers to a compound in which one molecule, such as an antibody, is effectively coupled to another molecule, such as a nanoparticle, either directly or indirectly by an appropriate means. In some embodiments, the molecule, preferably an antibody, may be directly covalently coupled to a nanoparticle, such as through a metal-thiol bond. In some embodiments, the molecule, preferably an antibody, may be coupled through the use of a "linker" molecule, as long as the linker does not substantially interfere with the function of the molecule, preferably the antibody. Preferably, the linker is biocompatible. Known molecular linkers include a maleimide or succinimide group, streptavidin, neutravidin, biotin, or similar compounds.

"Conjugating", "linking", "binding", or "linking" refers to coupling a first moiety to a second moiety comprises, but is not limited to, covalent bonding of one molecule to another (e.g., directly or via a linker molecule), non-covalent bonding of one molecule to another (e.g., electrostatic bonding, electrostatic conjugation), the non-covalent bonding of one molecule to another by hydrogen bonding, the non-covalent bonding of one molecule to another by van der Waals forces, and all combinations of such couplings. A skilled person knows methods for conjugating, linking, binding, or linking: coupling a first moiety to a second moiety covalent bonding, linking, or non-covalent bonding, and is able to apply them from the prior art in the meaning of the invention. "Direct linkage", as used herein, is understood as coupling or conjugation of two molecules without an intervening linker molecule. In some embodiments, a direct linkage is formed when an atom of a first molecule binds to an atom of a second molecule. In some embodiments, a direct linkage is formed when an atom of an antibody binds to an atom of a nanoparticle.. In some embodiments, the direct linkage is a covalent bond, such as a metal-thiol bond, preferably a gold-thiol bond.

The term "colocalize", as used herein, refers to occurring at the same or substantially the same location. In some examples, a metal precipitate (e.g., metal in oxidation state 0) formed by the methods described herein colocalizes with a target molecule when it accumulates or aggregates within at least about 5 µm of the target molecule (such as within at least about 1 µm, 500 nm, 250 nm, 100 nm, 50 nm, 20 nm, 10 nm, 5 nm, 2 nm, 1 nm, or 0.5 nm of the target molecule).

The term "contacting", as used herein, refers to placement that allows association between two or more entities, especially direct physical association, for example, in solid and/or liquid form (e.g., placing a biological sample or molecule, preferably an antigen attached to a filter or solved in a solvent, in contact with an antibody).

The term "detect" or "detecting", as used herein, is understood as to determine whether a target molecule, (e.g., an antigen) is present or absent in a sample, such as an aerosol exhaled from a human subject. In some examples, this may include quantification. "Detect" or "detecting" also refers to any method of determining whether or not something is present, such as determining whether a target molecule is present in a biological sample, such as an aerosol exhaled from a human subject. The term "detection" may include, for example, using a visual, acoustic and/or mechanical device to determine whether a sample has a particular characteristic, such as an antigen is present in an aerosol exhaled from a human subject. In some embodiments, detection comprises visual and/or acoustic observation that an antibody is bound to a target molecule, particularly an antigen, or the observation that an antibody is not bound to a target molecule, particularly an antigen.

"Specific binding" is to be understood as via one skilled in the art, whereby the skilled person is clearly aware of various experimental procedures that can be used to test binding and binding specificity. Some cross-reaction or background binding may be inevitable in many protein-protein interactions; this is not to detract from the "specificity" of the binding between antibody and epitope. The term "directed against" is also applicable when considering the term "specificity" in understanding the interaction between antibody and epitope.

An "antigen" refers to any target particle or molecular structure that is recognized by an antibody. The antigen may be an antigen of a pathogen. The antigen may be a soluble antigens. In one embodiment, the antigen may be a nucleic acids, preferably nucleic acid of a pathogen. In one embodiment, the antigen may be a toxin, preferably a toxin of a pathogen. In one embodiment, antigen may be from a blood borne pathogen or bacteria or viruses or the like, wherein bacteria and viruses are preferably pathogens.

The antigen can be a protein, a peptide, carbohydrates, a lipid or a nucleic acid, preferably from a pathogen.

The antigen is also a antigen of a human pathogen, such as a viral antigen, fungal antigen or a bacterial antigen, causing infectious disease, such as COVID-19, respiratory syndrome, pertussis, pneumonia, or tuberculosis.

### Transmission

The term "transmission" of pathogens, as used herein, is also understood to mean "contagion," "entry," "transfer," and "infection," preferably the transmission of viruses into cells of humans. The person skilled in the art knows that the use of human pathogenic viruses and virus components outside of laboratories to measure their spread and transmission using a device of the invention is an unacceptable hazard. In particular, the hazard may exist in the immediate temporal context as well as days and weeks after the measurement. Such measurement is also prohibited by the Biological Substances Ordinance. Aerosol particles can also be used, in particular to simulate the spread and transmission of viruses, preferably for coronaviruses, even more preferably SARS-CoV-2.

### Sample

The term "sample" refers to any liquid, semisolid, or solid substance (or material) in or on which a target may be present. In particular, a sample may be a biological sample. Examples of biological samples include saliva and aerosols exhaled from a human subject. In some embodiments, the biological sample is obtained from a human subject.

A biological sample includes any solid or liquid sample obtained from, excreted by, or exhaled by a living organism, particularly samples from a healthy or apparently healthy human subject or a human patient affected by a condition or disease to be diagnosed or investigated, such as SARS-CoV-2. For example, a biological sample may be aerosols produced by a human subject or saliva. In one embodiment, a sample is a quality control sample. In one embodiment, a sample is a test sample. For example, a test sample is a molecule (e.g., antigen) obtained from a pathogen. In one embodiment, the subject is a person at risk for or who has acquired a particular condition or disease.

The present invention, using "aerosols from exhaled air" and other non-invasively collected samples, provides a simple, non-invasive method for performing antigen detection, preferably SARS-CoV2 detection. "Non-invasive", as used herein, generally refers to a device or technique that requires minimal or no insertion into an opening, and that causes no significant discomfort to the patient. Non-invasive collection of respiratory specimens refers to collection methods that do not involve inserting an object into the patient's nose, mouth, or throat. Non-invasive collection methods can usually be performed by an individual without special medical training, but minimal assistance may be required for young children and infants. In one embodiment, the method disclosed herein comprise non-invasive sampling.

### Multiplex

Samples can include multiple targets, i.e. antigen, that can be specifically bound by an antibody. The simplest way to detect multiple targets is to use multiple antibodies bound to different nanoparticle having different wave spectra by increasing the number of detection channels. The system provides means to exploit antibody diversity for detection of multiple and rare target particles or antigens.

In some embodiments, multiple detectable nanoparticle that can be separately detected can be conjugated to different specific antibodies that specifically bind different antigens to provide a multiplexed assay that can provide detection of multiple pathogens in a sample.

In one embodiment, the solvent comprises a mixture of two or more different antibodies, wherein each different antibody is bound to a nanoparticle comprising a specific wave range of extinction and emission. In one embodiment, the solvent comprises a mixture of two or more different antibodies detecting 1 or 2 or 3 or 4 or 5 or 6 or 7 or 8 different antigens, wherein said antigens are preferably from different pathogens. The system described herein, allow detection of more than one different antigen, also referred to as "multiplex".

In some embodiments, the present disclosure provides multiplexed assays that allow separate detection, e.g., through multiple nanoparticles of different wave spectra, with a light sensor comprising 1, 2, 3, 4, 5, 6, 7 or 8 channels that allows for multiplexed analysis.

### Nanoparticle, metal ions

The term "nanoparticle", as used herein, refers to a nanoscale particle having a size measured in nanometers, such as a nanoscopic particle having at least one size of less than about 500 nm or less than about 400nm or less than about 300nm or less than about 200nm or less than about 100nm or less than about 50nm. Examples of nanoparticles include, but without limitation, metal nanoparticles, magnetic nanoparticles, paramagnetic nanoparticles, superparamagnetic nanoparticles, fullerene-like materials, inorganic nanotubes, dendrimers, such as with covalently bonded metal chelates, nanofibers, nanohorns, nano-onions, nanorods, nanoropes and quantum dots. In preferred embodiments, a nanoparticle is a metal nanoparticle, such as a nanoparticle of gold, palladium, platinum, silver, copper, nickel, cobalt, iridium, or an alloy of two or more of these metals. Nanoparticles may have a core or a core and shell, as in core-shell nanoparticles. In one embodiment, the nanoparticle is a gold nanoparticle. In one embodiment, the nanoparticle is a silver nanoparticle. In one embodiment, the nanoparticle is a platinum nanoparticle.

In one embodiment, sizes of nanoparticle range from 15 nm to 25 nm in diameter. In one embodiment, sizes of nanorods range from 15 nm to 25 nm in diameter and from 30 nm to 60 nm in length.

"Metal ions" are cations that require reduction and electrons for conversion to metal (zero oxidation state). Metal ions include, without limitation, gold, gold, copper, nickel, platinum, palladium, cobalt, or iridium ions. The metal ions may also comprise transition metal ions, such as from titanium. The metal ions may be in the form of a solution of a metal salt, such as a metal nitrate, metal halide, metal acetate, or metal perchlorate (e.g., silver nitrate, silver acetate, silver fluoride, or silver perchlorate). In other examples, the metal salt may be a metal sulfite, metal phosphate, or metal carbonate.

In one embodiment, antibody-nanoparticle conjugates are used. The antibody-nanoparticle conjugates can be used in methods for detecting a target molecule, such as a protein, a lipid, a carbohydrate or a nucleic acid molecule.

### Antibody-nanoparticle conjugates

The antibody-nanoparticle conjugates used herein comprise one or more nanoparticles, e.g., 1, 2, 3, 4, 5, 6, 7, 8, 9, 10 nanoparticles, or more nanoparticles, e.g., 2 to 10 nanoparticles, or 2 to 5 nanoparticles directly linked to an antibody by a metal-thiol bond between the nanoparticle and a thiol (e.g., an amino acid residue of the antibody, e.g., a cysteine residue) present on the antibody.

In some embodiments, the nanoparticles used in the antibody-nanoparticle conjugates are metallic nanoparticles. In some embodiments, the nanoparticles are gold, palladium, platinum, silver, copper, nickel, cobalt, or iridium, ruthenium, rhodium, osmium, or iron, preferably gold. In other examples, the nanoparticles are an alloy of two or more metals, for example, two or more gold, palladium, platinum, silver, copper, nickel, cobalt, or iridium metals, preferably gold and silver. In some embodiments, the nanoparticle is a core-shell nanoparticle having a metal core and a shell of another metal, preferably a silver nanoparticle having a gold shell. In some embodiments, the nanoparticle has a metal core with about 10-200 atoms, for example, about 100-200, 100-150, 11-100, or 11-70 atoms.

In one embodiment, the nanoparticle is a gold nanoparticle. Metallic nanoparticles and methods for producing metallic nanoparticles are well known in the art. See, e.g., Nanoparticles: From Theory to Application, Gunther Schmid, ed., Wiley-BCH, 2004.

In some embodiments, the two or more nanoparticles conjugated with an antibody each have a diameter of about 0.5 nm to about 200 nm, for example, about 1 nm to about 100 nm, about 2 nm to about 50 nm, about 2 nm to about 10 nm, or about 0.5 nm to about 50 nm. In certain examples, the nanoparticles have a diameter of about 5 nm or less, for example, about 5 nm, about 4.5 nm, about 4 nm, about 3.5 nm, about 3 nm, about 2.5 nm, about 2 nm, about 1.5 nm, about 1 nm, or about 0.5 nm or less. In other examples, the nanoparticles have a diameter of at least about 50 nm, such as about 60 nm, about 70 nm, about 80 nm, about 90 nm, about 100 nm, about 110 nm, about 120 nm, about 130 nm, about 140 nm, about 150 nm, about 160 nm, about 170 nm, about 180 nm, about 190 nm, about 200 nm, or more.

The conjugates used comprise two or more nanoparticles associated with an antibody. The antibody can comprise monoclonal or polyclonal antibodies, such as IgA, IgD, IgE, IgG, or IgM; Antibody fragments, including, without limitation, proteolytic antibody fragments (such as F(ab')2 fragments, Fab' fragments, Fab'-SH fragments, and Fab fragments as known in the art), recombinant antibody fragments (such as sFv fragments, dsFv fragments, bispecific sFv fragments, bispecific dsFv fragments, F(ab)'2 fragments, single-chain Fv proteins ("scFv"), and disulfide-stabilized Fv proteins ("dsFv")). In other examples, the antibody may comprise diabodies, genetically engineered antibodies, heteroconjugated antibodies (such as bispecific antibodies), and combinations thereof. In one embodiment, the antibody is a monoclonal antibody recognizing Spike protein of a coronavirus, preferably Spike protein of SARS-CoV, more preferably Spike protein of SARS-CoV-2. In one embodiment, the antibody is a monoclonal antibody recognizing nucleocapsid protein of a coronavirus, preferably nucleocapsid protein of SARS-CoV, more preferably nucleocapsid protein of SARS-CoV-2. In one embodiment, the antibody is a polyclonal antibody recognizing Spike protein of a coronavirus, preferably Spike protein of SARS-CoV, more preferably Spike protein of SARS-CoV-2. In one embodiment, the antibody is a polyclonal antibody recognizing nucleocapsid protein of a coronavirus, preferably nucleocapsid protein of SARS-CoV, more preferably nucleocapsid protein of SARS-CoV-2.

In one embodiment, the antibody-nanoparticle conjugates include a bond that directly links the antibody and the nanoparticle, such as a bond formed when an atom of a first molecule, an antibody, binds to an atom of a second molecule, a nanoparticle.

An aptamer is a short oligomer that forms a specific bond with a target substance with high affinity by forming a stable tertiary structure, and can be mass produced in a short time and at low cost using a chemical synthesis technique. Moreover, it is very stable to pH and temperature of the environment, so its potential for use in various fields such as environmental and medical diagnostics, e.g., detection of target substances and development of sensors for pathogen detection, is highly evaluated.

In one embodiment, an aptamer is bound to gold nanoparticles, wherein the aptamer has excellent in vivo stability and can specifically bind an antibody to be used for pathogen detection and infectious disease diagnosis.

A skilled person knows how to produce an antibody-nanoparticle conjugate suitable for the system and method described herein. Suitable methods for producing an antibody-nanoparticle conjugate are provided in the prior art.

### Gold nanoparticle under different coating conditions

To produce an antibody-nanoparticle conjugate suitable coating conditions for the system and method described herein, are known and described in the prior art, e.g. Pramanik A, Gao Y, Patibandla S, Mitra D, McCandless MG, Fassero LA, Gates K, Tandon R, Chandra Ray P. The rapid diagnosis and effective inhibition of coronavirus using spike antibody attached gold nanoparticles. Nanoscale Adv. 2021 Jan 18;3(6):1588-1596.

Certain examples for coating GNPs with antibodies are described below but should not be understood as limited to it. Other methods are also possible to achieve the goal of an optimal and functional GNP.

### Citrate coated gold nanoparticle synthesis

Citrate coated gold nanoparticles (GNPs) were synthesized using HAuCl4·3H2O and sodium citrate. For this purpose, we used a 0.01% solution of HAuCl4·3H2O and 1 wt% of sodium citrate. Transmission electron microscopy (TEM), and colorimetric and dynamic light scattering (DLS) data were used to characterize the freshly synthesized nanoparticles. The color of freshly prepared GNPs may be pink. GNPs exhibit unique optical properties due to surface plasmon resonance (SPR). It is well documented that the SPR band for citrate coated gold nanoparticles is due to phase changes resulting from the increased rate of electron-surface collisions compared with larger particles. The absorption spectra of citrate coated gold nanoparticles with λmax is seen to be ~520 nm. SPR of gold nanoparticles yields exceptionally high absorption, with extinction coefficient ε(15)520nm = 3.6 x 108 cm-1 M-1, which can be used for sensing of COVID-19 antigen or virus using naked eye colorimetric study.

The X-ray diffraction (XRD) spectrum from citrate coated gold nanoparticles. The particle concentration was measured by UV-visible spectroscopy using the molar extinction coefficient at the wavelength of the maximum absorption of the gold colloid (ε(15)519nm = 3.6 x 108 cm-1 M-1).

### PEG coated gold nanoparticle synthesis

To render the GNPs biocompatible, they can be coated with HS-PEG-COOH. For this purpose, carboxy-PEG12-thiol (HS-PEG12-COOH) can be used. 10 mg of HS-PEG12-COOH can be dissolved in 5 mL of water and citrate coated GNPs can be added using a syringe and vigorous stirring. After sonication of the mixture for 30 minutes, the final products can be separated through centrifugation. After PEG coating, the size of the GNPs can increase to ~18 ± 4 nm. The color of freshly prepared PEG coated GNPs may be pink. The absorption spectrum of PEG coated gold nanoparticles with λmax is seen to be ~521 nm, that may indicate the optical spectrum for GNPs.

### Anti-spike antibody coated gold nanoparticle synthesis

Anti-spike antibody can be attached to gold nanoparticles. EDC (1-ethyl-3-(3-dimethylaminopropyl)-carbodiimide)/NHS (N-hydroxysuccinimide) chemistry can be used. 0.2 molar (M) EDC and 0.05 M N-hydroxysulfosuccinimide sodium salt [1:3 (v/v) ratio] can be added to a solution containing GNPs and anti-spike antibody, and the mixture can be sonicated for 30 minutes. Anti-spike antibody attached GNPs can be separated from GNPs without antibody using centrifugation for 15 minutes at 6000 rpm, followed by resuspension in buffer. After antibody attachment, the size of GNPs can increase to ~27 ± 6 nm. The number of antibodies attached to the GNPs can be determined by synthesizing Cy3 dye attached anti-spike antibody conjugated GNPs. After separation of the Cy3 dye attached antibody conjugated GNPs from unconjugated molecules, 10 µM potassium cyanide can be added for oxidization of the GNPs and number of antibodies attached on GNPs can be estimated from fluorescence data, such as 10 ng mL-1 antibodies attached on GNPs.

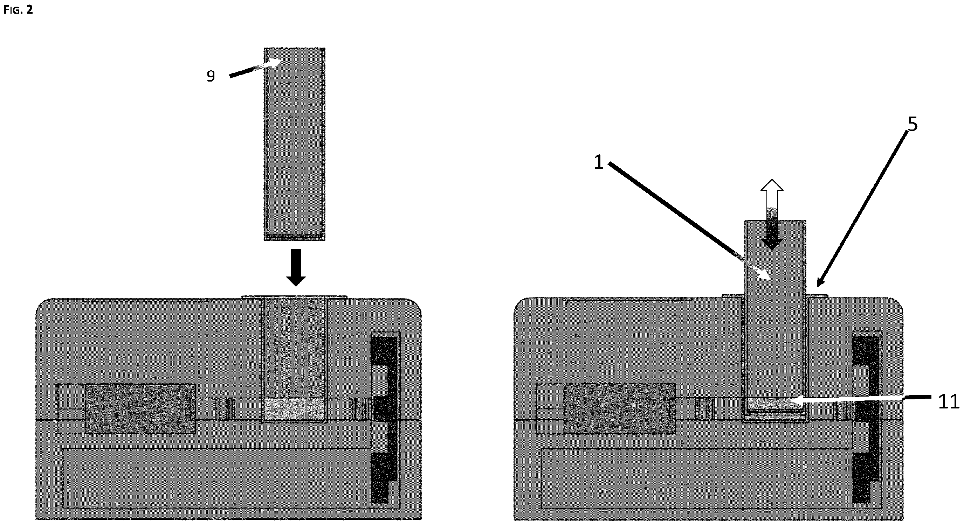

The color of freshly prepared antibody GNPs may be pink. Fig. 2D shows the absorption spectra of antibody attached gold nanoparticles with λmax is seen to be ~522 nm, that may indicate the optical spectrum for GNPs.

### Solvent

The term "solvent", as used herein, refers to a solution, for example an aqueous solution, suitable for solving an antigen and specifically binding of an antibody to said antigen. The solvent provides the optimal reaction conditions, including pH, for the antibody to be bound and remain bound to the nanoparticle and for the antibody to specifically recognize and optimally bind to the antigen. The solvent comprises the nanoparticle bound to the antibody in water, and one or more of the components including salts, detergents, blocking agent, reducing agent, anti-foaming agent, stabilizing agent and/or carbohydrates.

Various additives, concentrations and combinations thereof, suitable for the solvent are described herein as examples and are not intended to be limiting thereto. The prior art and one skilled in the art are aware of a variety of possible compositions for such solvents suitable to provide optimal conditions for antibody-antigen recognition, binding and color change. One skilled in the art can certainly select from the solvents in the prior art the appropriate additives, combinations and concentrations suitable for the system and method of the disclosed invention.

The term "reducing agent" refers to an element or compound that reduces another molecule or compound. When another molecule or compound is reduced, the reducing agent is oxidized and is an electron donor. Specific examples of reducing agents include, but are not limited to, dithiothreitol (DTT), beta mercaptoethanol, and sodium thiosulfate.

A "carbohydrate" is also a polysaccharide. In one embodiment, the solvent comprises a polysaccharide at a final concentration of about 0.5% to about 25% wt / vol. In one embodiment, the solution comprises the polysaccharide at a final concentration of about 1% to about 20% wt / vol. In one embodiment, the solution comprises the polysaccharide at a final concentration of about 2% to about 15% wt / vol. In one embodiment, the solution comprises about 2%, 3%, 4%, 5%, 6%, 7%, 8%, 9%, 10%, 15%, 20%, 25%, 30% or more, including all values in between. In some embodiments, the sensitivity of method described herein improves by using polysaccharide, e.g., sucrose, trehalose, maltodextrin, sorbitol, mannitol or ficoll. One embodiment, the polysaccharide is ficoll. In yet another exemplary embodiment, the polysaccharide is dextran.

A "blocking agent, used herein, is understood to prevent non-specific binding of antibodies to antigens. In one embodiment, the solvent comprises a blocking agent at a final concentration of about 0.1% to about 20% wt / vol. In another embodiment, the solution comprises a blocking agent at a final concentration of about 0.5% to about 10% wt / vol. In yet another embodiment, the solution comprises a blocking agent at a final concentration of about 1% to about 5% wt / vol. In an exemplary embodiment, the solution comprises a blocking agent at a final concentration of about 1%, 2%, 3%, 4%, or 5%, including all values in between. In various embodiments described herein, the sensitivity of the assay may be improved when the blocking agent is added to the solution when compared to an assay performed in the absence of the blocking agent. In some embodiments, the blocking agent is selected from bovine serum albumin, casein, gelatin, ovalbumin, and gamma globulin. In an exemplary embodiment, the blocking agent is bovine serum albumin (BSA).

In some embodiments, the solvent comprises one or more of maltodextrin, trehalose, PEG, a blocking agent (e.g., BSA), and/or sodium chloride. In an exemplary embodiment, one or more of the solution components, e.g., maltodextrin, is a lyophilized bead or pellet that is suspended upon addition of a liquid, e.g., water, saline, or a liquid biological sample. It may be provided as. For example, one or more of the solution components may be provided as beads in a spectrophotometric cuvette or a reaction chamber of an analyzer rotor that are suspended in the solution upon addition of the liquid.

In addition, salts, such as chaotropic salts, can have an effect on gold conjugate colloid stability. In some embodiments, the solvent comprises a salt. Salts are known by a person skilled in the art as chemical compounds composed of positively charged ions, called cations, and negatively charged ions, called anions, and that ionic bonds exist between these ions. Cations in the sense of the invention can be, among others, sodium, potassium, calcium, magnesium or lithium, preferably sodium. Anions in the sense of the invention can be, among others, chloride, iodide, sulfate, nitrate, acetate, succinate or citrate ions, preferably chloride ions. In some embodiments, the salt, NaCl, MgCI2, CaCI2, NaSCN, KCI, sodium phosphate, Tris-HCI, . In some embodiments, the chelating agent is selected from the group consisting of ethylenediaminetetraacetic acid (EDTA) and ethylene glycol bis (β-aminoethyl ether) -N, N, N ', N'-tetraacetic acid (EGTA). In one embodiment, the solvent comprises EDTA or EGTA. Salts are usually used in form of a buffer in solvents. For example, solvents comprise the buffer Phosphate-buffered saline (PBS) or HEPES or Tris(hydroxymethyl)aminomethan (TRIS; e.g., TRIS-HCI) or Imidazole (e.g., Imidazole-HCI) are used. Method of adding a phosphate buffer, sodium chloride and the like are known (Japanese Published Unexamined Patent Application No. 504499/91). Buffer systems suitable for an optimal antibody-antigen recognition and binding are known on the prior art (e.g. Antibody composition and buffer system therefor WO2014191560A1)

A "detergent" is a surfactant or a mixture of surfactants. Detergents comprise sodium salts of long chain alkyl hydrogen sulphate or a long chain of benzene sulphonic acid., Detergents haven an effect on antigen-antibody interaction, reduce background signals, and promote specific antibody binding. Certain suitable detergens, and general concentrations of such detergents, are described herein, but it is known by the skilled person that other detergents and concentrations can be used. One skilled in the art would select detergents and concentrations available in the prior art suitable for the method and system of the disclosed invention. (M.J. CRUMPTON and R.M.E. PARKHOUSE, FEBS LETTERS, Volume 22, number 2, May 1972, p210-212)

Detergents used in solvents for antigen-antibody binding comprise, for example, sodium dodecyl sulphate (SDS, in the range between 0.01% and 4%w/v), TritonX-100 (in the range between 0.01% and 4%w/v), Tween20 (in the range between 0.1% and 5%w/v), Tween80 (in the range between 0.1% and 45%w/v), Nonidet P40 (in the range between 0.1% and 5%w/v), Brji 58 between (in the range between 0.1% and 5%w/v), deoxycholic acid sodium salt (DOC, in the range between 0.1% and 4%w/v), cetyltrimethylammonium bromide (CTAB, in the range between 0.1% and 4%w/v), cholic acid (in the range between 0.1% and 4%w/v), dioctyl sulfosuccinate (in the range between 0.1% and 3%w/v), or N-lauroylsarcosine (in the range between 0.01% and 2%w/v). The solvent may comprise a detergent at a final concentration (w/v) of 0.01%, 0.05%, 0.1%, 0.2%, 0.3%, 0.4%, 0.5%, 0.6%, 0.7%, 0.8%, 0.9%, 1%, 1.5%, 2%, 2.5%, 3%, 35%, 4%, 4.5% or 5 %, and any value in between. In one embodiment, 1% SDS or less is used. In one embodiment, 0.5% SDS or less is used. In one embodiment, 0.2% SDS or less is used. In one embodiment, 0.01% SDS or less is used. In one embodiment, 1% N-lauroylsarcosine or less is used. In one embodiment, 0.5% N-lauroylsarcosine or less is used. In one embodiment, 0.1% N-lauroylsarcosine or less is used. In one embodiment, 0.01% N-lauroylsarcosine or less is used. In one embodiment, 1% Tween20 or less is used. In one embodiment, 0.5% Tween20 or less is used. In one embodiment, 0.1% Tween20 or less is used. In one embodiment, 0.01% Tween20 or less is used.

A "defoamer" or "anti-foaming agent" refers to a chemical additive that reduces and prevents the formation of foam. The anti-foaming agent can be selected from, but not limited to, mineral oil, vegetable oil, white oil or any other oil that is insoluble in the foaming medium, waxes, ethylene bis stearamide (EBS), paraffin waxes, ester waxes, fatty alcohol waxes, powder defoamers, water based defoamers, silicone based defoamers. For example, anti-foams are added in certain types of detergents to reduce foaming that might decrease the action of the detergent. In one embodiment, the solvent comprises

A "stabilizing agent" refers to chemical compounds added to a metastable system to prevent its transition to a lower-energy state. In solvents as decribed herein, a stabilizing agent helps to prevent antibody degradation and to minimize protein aggregation. For example, the stabilizing agents N-Acetylcysteine or BSA or citric acid or sodium azide, or glycine (also glycerol) or urea or imidazole, are added to solvents comprising antibodies.

The amount of glycine to be added may be any concentration as long as the glycine concentration is in the range of 10 to 30 mg/mL. Examples of the form of glycine to be added include glycine, pharmaceutically acceptable salts of glycine such as glycine hydrochloride and the like. In one embodiment, the solvent comprises glycine at 20 to 25 mg/mL, more preferably 22 to 23 mg/mL. The amount of citric acid to be added may be any concentration as long as the citric acid concentration is in the range of 0.1 to 50 mmol/L. Examples of the form of citric acid to be added include citric acid, pharmaceutically acceptable salts of citric acid such as sodium citrate and the like. In one embodiment, the solvent comprises citric acid at 0.5 to 20 mmol/L, preferably 1 to 10 mmol/L.

The amount of N-Acetylcysteine to be added may be any concentration as long as the N-Acetylcysteine is in the range of 0.1 to 50 mM. Examples of the form of N-Acetylcysteine to be added include N-Acetylcysteine, pharmaceutically acceptable salts of N-Acetylcysteine such as N-Acetylcysteine salt and the like. In one embodiment, the solvent comprises N-Acetylcysteine at 0.5 to 20 mM, preferably 1 to 10 mM.

The amount of BSA to be added may be any concentration as long as the BSA concentration is in the range of 0.1-20mg/ml. In one embodiment, the solvent comprises BSA at 0.5-10mg/ml, preferably 1 to 5 mg/ml.

The amount of sodium azide to be added may be any concentration as long as the sodium azide concentration is in the range of 0.005-0.2% (w/v). Examples of the form of sodium azide to be added include sodium azide, pharmaceutically acceptable salts of sodium azide. In one embodiment, the solvent comprises sodium azide at 0.01-0.1% (w/v), preferably 0.02-0.05% (w/v).

The amount of urea to be added may be any concentration as long as the urea concentration is in the range of 0.1-20% (w/v). Examples of the form of urea to be added include urea pharmaceutically acceptable salts of urea. In one embodiment, the solvent comprises urea at 1-10% (w/v), preferably 2.5-7.5% (w/v).

The solvent has a pH between 4 and 8, such as pH 4 or more, pH 5 or more, pH 6 or more, pH 7 or more or pH 8 or more, and all values in between. In one embodiment, the pH of the solvent ranges between pH 6.5 and 7.5, preferably between pH 6.8 and pH 7.3, more preferably between pH 6.9 and pH 7.1.

In one embodiment, the solvent can comprise 100 mM HEPES at pH 7.5; 50 mM NaCl; 0.5 % (w/v) Saccharose; 70 mM Urea, 3 mM N-Acetylcysteine; 1 mM EDTA, and 0.1 % (w/v) Tween20.

In one embodiment, the solvent can comprise 200 mM HEPES at pH 7.5; 100 mM NaCl; 1 % (w/v) Saccharose; 140 mM Urea, 6 mM N-Acetylcysteine; 2 mM EDTA, and 0.1 % (w/v) Tween20.

### Filter

The "filter", as used herein, refers to any material providing porous or fibrous matrix to which an antigen can reversibly bind or reversible attach and that filters based on physical absorption in the matrix channels, e.g., size, solubility, or charge. The matrix may have a thickness of 0.1mm, 0.2mm, 0.3mm, 0.4mm, 0.5mm, 1mm, 2mm, 3mm, 4mm, 5mm or more. In one embodiment, the filter is a size filter. The filter is mounted directly to the first container. In one embodiment, the filter has hydrophilic properties. In one embodiment, the filter has hydrophilic properties.

In one embodiment, the filter is a charged filter. The charged filter comprises a porous matrix that filters molecules and/or solutions based on electro-kinetic adsorption, e.g., based on a charge on the matrix. A variety of positively charged ions, preferably metal ions, are suitable for use in such a filter. In some embodiments, a charged filter comprises at least one diatomaceous earth layer and/or a positively charged ion, preferably a metal ion.

In one embodiment, the filter is a size filter. The size filter, also a retention filter, comprises material having a porous matrix, wherein the matrix channels is suitable for retention of any antigen larger in size than the maximum diameter of the matrix channels and for the antigen to retain on the material, preferably the antigen from an aerosol of a human subject. Any antigen smaller in size than the maximum diameter of the matrix channels can pass through the matrix channels. In one embodiment, the maximum diameter of the matrix channels is suitable for retention an antigen used for detecting a specific pathogen. Another type of filters are membrane filters that retain particles, such as an antigen, on the surface. "Retention" refers to the capacity of the filter to retain particles of a given size.

In the context of the present invention, the matrix of filter selected, is able to bind and retain the antigen during the step of collecting antigens from aerosols of a human subject and releasing said antigen during the subsequent dispensing steps and maintain them in a form such that an antibody can recognize said antigen.

Such filter can be penetrated by the solvent. The matrix should also be a suitable material for reversible capture of the scavenged antigen. In one embodiment, the method comprises contacting one or more filters to an aqueous solvent comprising antigen-binding protein molecules (antibody) bound to nanoparticles. In one embodiment, contacting said filters to the solvent leads to a release of antigens captured by said filters into said solvent.

In one embodiment, the filter is completely covered by the solvent for releasing antigens into the solvent. Releasing antigens into the solvents comprise pressing, mixing, flushing, and/or up and down movement of the filter in the solvent. Releasing antigens into the solvent also means dispensing or intermingling. In one embodiment, the filter is flushed in the solvent. In one embodiment, the filter is pressed into the solvent. In one embodiment, antigens are released and dispensed into the solvent by flushing the filter in the solvent. In one embodiment, antigens are released and dispensed into the solvent by pressing the filter in the solvent. In one embodiment, antigens are released and dispensed into the solvent by up and down movement of the filter in the solvent.

Certain suitable materials, and general properties of such materials, are described herein, but it is known by the skilled person that other materials can be used. One skilled in the art would select filters available in the prior art suitable for the method and system of the disclosed invention.

In one embodiment, the filter material is FFP2 filter material. In one embodiment, the filter material is FFP3 filter material.

Materials used for filters can be selected from cellulose, quartz, polymers, and glass. In one embodiment, the filters material is cellulose and is preferably unmodified. Filters are often referred to in terms of target particle size retention, such as very fine particles, e.g. from several micrometers (e.g. 10 µηι or less, 5 µηι or less, or 2 µηι. or less) to sub-micrometer size range (e.g. down to 0.2 µηι or even 0.1 µηι in size). The pore diameter may be in the range 0.01µm to 100µm or in the range 0.02 µm to 50 µm or in the range 0.05µm to 20µm or in the range 0.075µm to 1 µm or in the range 0.1µm to 0.75 µm or in the range 0.1µm to 0.3 µm. Fibrous materials for filters can be selected from, but not limited to, cellulose, quartz, polymers, and glass, asbestos, potassium titanate, aluminum silicate, mineral wool, regenerated cellulose, polyvinyl chloride, polyvinylidene chloride, polyacrylonitrile, polyethylene, polypropylene, rubber, polymers of *terephthalic acid and ethylene glycol, polyamides, casein fibers, zein fibers, cellulose acetate, viscose rayon, hemp, jute, linen, cot-ten, silk, wool, mohair, paper, and metallic fibers. Particulate materials such as diatomaceous earth, fullers earth, silicon, magnesia, silica, talc, silica gel, alumina, quartz, carbon, activated carbon, clays, synthetic resins and cellulose derivatives, such as polyethylene, polystyrene, olypropylene, urea-formaldehyde, phenol-formaldehyde, polytetra fiuorethylene, polytrifluorochloroethylene, polymers of terephthalic acid and ethylene glycol, polyacrylonitrile, ethyl cellulose, polyamides, and cellulose acetate-propionate, and metal particles such as aluminum, iron, copper, nickel, chromium, and titanium and metal alloys of all kinds, such as monel, brass, stainless steel, bronze, Inconel, cupronickel, Hastelloy, beryllium, and copper can also be employed. Combinations of fibers and particles are also useful, such as combinations of diatomaceous earth and glass fibers.

In one embodiment, the filter comprise a filter with a pore diameter of at least 0.1µm and a filter thickness of less than 0.5 mm.

In one embodiment, the filter comprise a filter with a pore diameter of at least 0.1µm and a filter thickness of less than 1 mm.

In one embodiment, the filter comprise a filter with a pore diameter of at least 0.5µm and a filter thickness of less than 0.5 mm.

In one embodiment, the filter comprise a filter with a pore diameter of at least 0.5µm and a filter thickness of less than 1 mm.

### FIGURES

The invention is demonstrated by way of the example through the figures disclosed herein. The figures provided represent particular, non-limiting embodiments and are not intended to limit the scope of the invention.

### Short description of the figures:

- **Figure 1:**: **Illustration of the Passive blow test system: Exterior view and Cross section**
- **Figure 2:**: **Illustration of the Passive blow test system: Cross section**
- **Figure 3:**: **Illustration of the Passive blow test system: Cross section of optical unit**

### Detailed description of the figures:

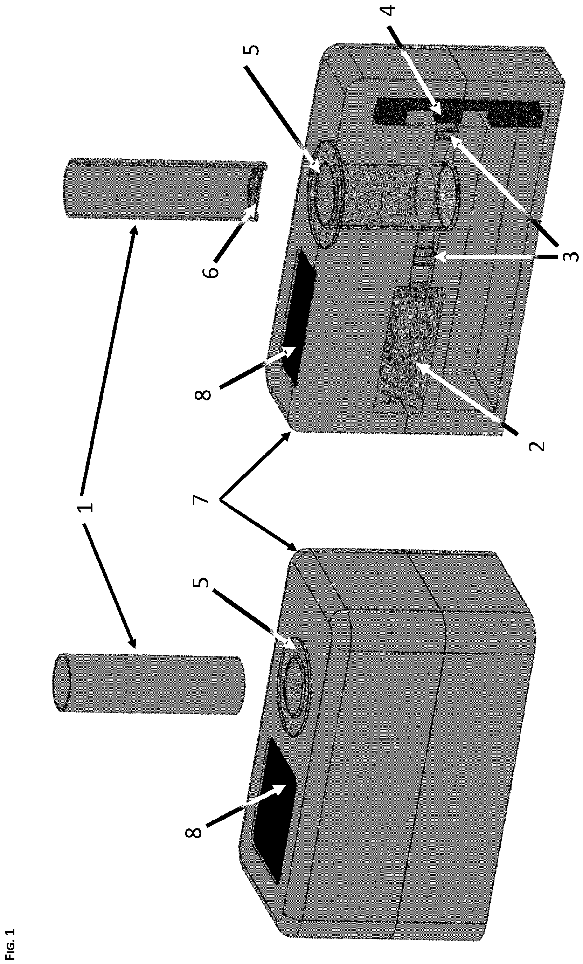

**Figure 1****: Illustration of the Passive blow test system: Exterior view and Cross section.** Exterior view (left) and Cross section (right) of evaluation unit (7) and collecting tube (first container) (1). The collecting tube or first container (1) features a filter (6) at one site. The evaluation unit incorporates a light source (2), a lens arrangement (3) a light sensor (4), a disposable fluid container (5) and user Interface (8)

**Figure 2****: Illustration of the Passive blow test system: Cross section.** Collecting principle: Left: Individuals breathe through the disposable collecting tube (1) at the site with the filter (6), so that aerosol particles in the breath attach to the filter (6). At the second site of the collecting tube (9), there might be a feature to control the amount of exhaled air, e.g. a whistle, spirometer or a baloon. Right: the collecting tube gets incorporated into the fluid container. A sealing between the shell surfaces of collecting tube and fluid container or second container (5) forces the fluid through the filter. A shuttling stroke motion between collecting tube or first container (1) and fluid container or second container (5) flushes the filter and dissolves collected aerosols out of the filter into the solvent.

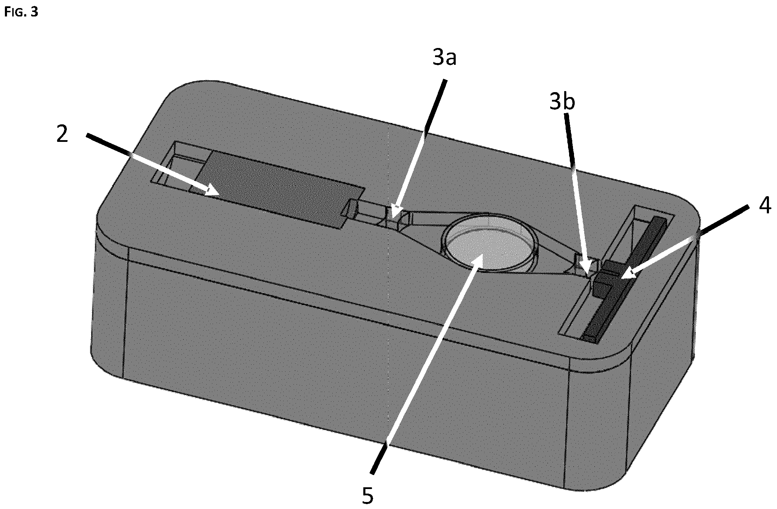

**Figure 3****: Illustration of the Passive blow test system: Cross section of optical unit.** Light from the light source (2) gets expanded by an optical lens (3a) to illuminate the fluid in the transparent container (5). The scattered and not absorbed light gets focused by the optical lens (3b) into the light sensor (4).

### EXAMPLES

The invention is demonstrated through the examples disclosed herein. The examples provided represent particular embodiments and are not intended to limit the scope of the invention. The examples are to be considered as providing a non-limiting illustration and technical support for carrying out the invention.

The examples below present the individual parts and structure passive blow test system.

### Example 1: Composition and structure of the system - Passive blow test

Individuals breathe through the disposable collecting tube at the site with the filter, so that aerosol particles in the breath attach to the filter. At the second site of the collecting tube, there might be a feature to control the amount of exhaled air, e.g. a whistle, spirometer or a baloon. After this, the collecting tube gets incorporated into the fluid container. A sealing between the shell surfaces of collecting tube and fluid container forces the fluid through the filter. A shuttling stroke motion between collecting tube and fluid container flushes the filter and dissolves collected aerosols out of the filter into the solvent. In case of the presents of specific antigens in the aerosols, the measuring fluid changes its optical properties which is detectable by the optical unit.

### REFERENCES

(1) Guglielmi, G. The Explosion of New Coronavirus Tests That Could Help to End the pandemic. Nature 2020, 583 (7817), 506- 509, DOI: 10.1038/d41586-020-02140-8
(2) Bhatraju, P. K.; Ghassemieh, B. J.; Nichols, M.; Kim, R.; Jerome, K. R.; Nalla, A. K.; Greninger, A. L.; Pipavath, S.; Wurfel, M. M.;Evans, L.; Kritek, P. A.; West, T. E.; Luks, A.; Gerbino, A.; Dale, C. R.; Goldman, J. D.; O'Mahony, S.; Mikacenic, C. COVID-19 in Critically III Patients in the Seattle Region Case Series. N. Engl. J. Med. 2020, 382, 2012-2022.
(3) Udugama, B.; Kadhiresan, P.; Kozlowski, H. N.; Malekjahani, A.; Osborne, M.; Li, V. Y. C.; Chen, H.; Mubareka, S.; Gubbay, J. B.; Chan, W. C. W. Diagnosing COVID-19: The Disease and Tools for Detection. ACS Nano 2020, 14, 3822-3835.
(4) Lassaunière, R.; Frische, A.; Harboe, Z. B.; Nielsen, A. C.; Fomsgaard, A.; Krogfelt, K. A.; Jørgensen, C. S. Evaluation of Nine Commercial SARS-CoV-2 Immunoassays. medRxiv 2020, 1, DOI: 10.1101/2020.04.09.20056325
(5) Borges, J.T., Nakada, L.Y.K., Maniero, M.G. et al. SARS-CoV-2: a systematic review of indoor air sampling for virus detection. Environ Sci Pollut Res 28, 40460-40473 (2021).
(6) Bhalla, N.; Pan, Y.; Yang, Z.; Payam, A. F. Opportunities and Challenges for Biosensors and Nanoscale Analytical Tools for Pandemics: COVID-19. ACS Nano 2020, 14, 7783.

## Claims

1. System for antigen detection comprising a first container (1) configured to collect aerosols out of exhaled air of a human subject comprising aerosols and a second container (5) suitable for a solvent, wherein nanoparticles are dissolved in the solvent and the nanoparticles are linked to antibodies, further wherein a change of optical properties of the solvent is detectable upon contact between the antibodies and matching antigens, wherein
- the system comprises a measuring cell and within the measuring cell there is a spectrometer comprising a light source (2) and a light sensor (4)
- and the first container comprises a hole at first side and a filter (6) at second side
- and the second container (5) is configured to incorporate the first container (1), wherein the filter (6) of the first container (1) contacts the solvent of the second container (5) in the measuring cell,
- wherein during incorporating of the second container (5) into the first container (1), a sealing between the shell surfaces of both containers forces the enclosed solvent in the second container (5) to get pushed through the filter (6) of the first container (1),
- an executable shuttling stroke motion between the first container (1) and the second container (5) flushes the filter and dissolves collected aerosols out of the filter into the solvent,
- wherein the light source (2) irradiates the solvent and the light sensor (4) is arranged to detect the change of optical properties
- and the system comprises an alarm device adapted to emit an alarm signal upon detection of the change of optical properties.

2. System according to one or more of the preceding claims
**wherein**
the system comprises a computing unit, wherein the computing unit is configured to calculate an concentration of an antigen based on the change of optical properties of the solvent.

3. System according to one or more of the preceding claims
**wherein**
the light source (2) is a monochromatic laser comprising linearly polarized light, wherein said linearly polarized light is configured to excite pathogen-nanoparticle-agglomerates at extinction peak-wavelength,
wherein said wavelengths preferably can be between 600 nm and 700nm, preferably between 610 nm and 690 nm, more preferably between 620 nm and 680 nm, even more preferably between 630 nm and 670 nm, even more between preferably 640 nm and 660 nm, even more preferably between 645 nm and 655 nm.

4. System according to one or more of the preceding claims
**wherein**
the alarm signal is selected from a group comprising a visual signal and/or an acoustic sign.

5. System according to one or more of the preceding claims
**wherein**
the nanoparticles comprise a material selected from a group comprising gold, silver, ruthenium, rhodium, palladium, osmium, iridium and/or platinum and/or
the nanoparticles might be spheres in the range of 15-25 nm or nanorods with a diameter of 15-25nm and a length of 30-60nm.

6. System according to one or more of the preceding claims
**wherein**
the filter (6) can be a hydrophilic filter and/or a hypophilic filter, preferably a filter suitable for reversibly attaching of the antigen.

7. System according to one or more of the preceding claims
**wherein**
the exhaled air of the human subject can be collected by direct contact of the mouth of said subject with the first container.

8. System according to one or more of the preceding claims
**wherein**
the solvent additionally comprises a salt, a detergent, an anti-foaming agent, a stabilizing agent, a blocking agent, and/or water.

9. System according to one or more of the preceding claims
**wherein**
the antibody is suitable to contact the antigen, wherein the antigen can be a protein, a peptide, carbohydrates, a lipid or a nucleic acid, preferably a peptide or a protein, more preferably a protein, wherein the antigen is a antigen of a human pathogen, such as a viral antigen, fungal antigen or a bacterial antigen, causing infectious disease, such as COVID-19, respiratory syndrome, pertussis, pneumonia, or tuberculosis.

10. System according to one or more of the preceding claims
**wherein**
the human pathogen is selected from a group of bacteria, fungi or viruses, such as a corona virus, influenza virus, mycobacteriaceae, streptococcae, preferably SARS corona virus, respiratory syndrome virus, streptococcus mutans, mycobacterium tuberculosis, streptococcus pneumoniae, more preferably SARS-CoV-2.

11. System according to one or more of the preceding claims
**wherein**
the viral antigen can be selected from, but not limited to, SARS-CoV-2 S-protein, SARS-CoV-2 N-protein, hPMV G protein, hPMV F protein, RSV F protein, RSV G protein, or Influenza A/B protein, such as HA, MI, M2 and/or NA.

12. System according to one or more of the preceding claims
**wherein**
the bacterial antigen comprises antigens including, but not limited to, pertussis-toxin, streptococcus pneumoniae C-polysaccharide, early secreted antigenic target, or culture filtrate protein.

13. A method using the system according to any of the preceding claims 1-12 for antigen detection in exhaled air of a human subject comprising aerosols,
**wherein**
the antigen detection comprises:
(a) Collecting antigens from aerosols of said human subject into the system according to claim 1-12, by direct contact of the mouth of said subject to the first end of a first container (1) and said container (1) comprising a filter (6) at the second end, and
(b) Attaching said antigens to the filter (6), wherein said attaching is reversible, and
(c) Dispensing said antigen into the solvent according to claims 1-12 by the filter (6) being completely covered by the solvent, and
(d) Contacting said antigen with the antibody, wherein the contact causes a change of optical properties of the solvent; and is preferably a spectral sensor
(e) Determining said change of optical properties by means of the light sensor (4) according to claim 3 and transferring it to computing unit according to claim 2, wherein said change of optical properties, and
(f) Calculating concentration of an antigen based on the change of optical properties of the solvent, wherein said concentration is a first parameter, and
wherein, said antigen detection comprises identifying at least one type of the human pathogen according to claim 10.

14. The method according to claim 13,
**wherein**
the first parameter is compared to a second parameter by the computing unit, and wherein the alarm device according to any one of claims 1-12 produces a visual and/or an acoustic signal when the first parameter is greater than or identical to the second parameter, wherein the second parameter is a pre-set value.

15. The method according to claim 13 or 14,
**wherein**
the antigen detection comprises identifying a human pathogen, preferably a virus, a fungi, or a bacteria, , more preferably corona virus, streptococcaceae, bordetella, mycobacteriaceae or influenza viruses, even more preferably SARS-CoV-2.
